# EUROPEAN PATENT SPECIFICATION

(11) **EP 4 334 297 B1**
(45) Date of publication and mention of the grant of the patent: **22.10.2025**
(21) Application number: 22727132.7
(22) Date of filing: 04.05.2022
(51) Int. Cl.: C07D 307/33

(54) **METHOD FOR PREPARING LACTONES HAVING AT LEAST ONE GROUP SELECTED FROM CARBOXYL, CARBOALKOXY, HYDROXY AND CARBOXYLATE**
VERFAHREN ZUR HERSTELLUNG VON LACTONEN MIT MINDESTENS EINER AUS CARBOXYL, CARBOALKOXY, HYDROXY UND CARBOXYLAT AUSGEWÄHLTEN GRUPPE
PROCÉDÉ DE PRÉPARATION DE LACTONES AYANT AU MOINS UN GROUPE SÉLECTIONNÉ PARMI LE CARBOXYLE, LE CARBOALKOXY, L'HYDROXY ET LE CARBOXYLATE

(30) Priority: 04.05.2021 EP 21172097
(43) Date of publication of application: 13.03.2024
(73) Proprietor: BASF SE, 67056 Ludwigshafen am Rhein (DE)
(72) Inventor: GROESSL, Sylvester, 67056 Ludwigshafen (DE); LINKE, Stephanie Sybille, 67056 Ludwigshafen (DE); BAIER, Grit, 67056 Ludwigshafen (DE); TAVARES ANDRE, Rute da Conceicao, 67056 Ludwigshafen (DE)
(74) Representative: BASF IP Association
(86) International application number: PCT/EP2022/061912
(87) International publication number: WO 2022/233906

(56) References cited:
- US-A- 4 218 381
- JASPER VERDUYCKT ET AL: "Stabilising Ni catalysts for the dehydration-decarboxylation-hydrogenation of citric acid to methylsuccinic acid", GREEN CHEMISTRY, vol. 19, no. 19, 1 January 2017 (2017-01-01), GB, pages 4642 - 4650, XP055423645, ISSN: 1463-9262, DOI: 10.1039/C7GC01773A
- XU P F ET AL: "A facile method for synthesis of (R)-(-)- and (S)-(+)-homocitric acid lactones and related @a-hydroxy dicarboxylic acids from d- or l-malic acid", TETRAHEDRON LETTERS, ELSEVIER, AMSTERDAM , NL, vol. 46, no. 22, 30 May 2005 (2005-05-30), pages 3815 - 3818, XP027864023, ISSN: 0040-4039, [retrieved on 20050530]
- THAPA INDIRA ET AL: "C6 Diacids from homocitric acid lactone using relay heterogeneous catalysis in water", CATALYSIS TODAY, vol. 319, 1 January 2019 (2019-01-01), AMSTERDAM, NL, pages 191 - 196, XP055846547, ISSN: 0920-5861, DOI: 10.1016/j.cattod.2018.08.002
- VENKATESWARA RAO G ET AL: "Hydroxycitric acid lactone and its salts: Preparation and appetite suppression studies", FOOD CHEMISTRY, ELSEVIER LTD, NL, vol. 120, no. 1, 1 May 2010 (2010-05-01), pages 235 - 239, XP026799346, ISSN: 0308-8146, [retrieved on 20091013]
- HIDA HIROYUKI ET AL: "Production of Hydroxycitric Acid by Microorganisms", BIOSCIENCE, BIOTECHNOLOGY, AND BIOCHEMISTRY, vol. 69, no. 8, 1 January 2005 (2005-01-01), pages 1555 - 1561, XP055846554, ISSN: 0916-8451, DOI: 10.1271/bbb.69.1555
- GÉRARDY ROMARIC ET AL: "Continuous-Flow Preparation of [gamma]-Butyrolactone Scaffolds from Renewable Fumaric and Itaconic Acids under Photosensitized Conditions", vol. 21, no. 12, 15 December 2017 (2017-12-15), US, pages 2012 - 2017, XP055844503, ISSN: 1083-6160, Retrieved from the Internet <URL:https://pubs.acs.org/doi/pdf/10.1021/acs.oprd.7b00314> DOI: 10.1021/acs.oprd.7b00314
- WALRAVEN H G M ET AL: "A facile two synthon approach to the camptothecin skeleton", TETRAHEDRON, ELSEVIER SIENCE PUBLISHERS, AMSTERDAM, NL, vol. 36, no. 2, 1 January 1980 (1980-01-01), pages 321 - 327, XP022809055, ISSN: 0040-4020, [retrieved on 19800101], DOI: 10.1016/0040-4020(80)80022-6

## Description

The present application relates to a method of preparing a product comprising (a) one or more product compounds selected from the group consisting of lactones having at least one group selected from carboxyl, carboalkoxy, hydroxy and carboxylate, and/or (b) one or more reaction products of one or more of said compounds selected from the group consisting of lactones having at least one group selected from carboxyl, carboalkoxy, hydroxy and carboxylate, to such products (a) and (b), and to the use of compounds selected from the group consisting of aliphatic tricarboxylic acids, esters thereof, anhydrides thereof, and salts thereof as starting compounds for making product compounds selected from the group consisting of lactones having at least one group selected from carboxyl, carboalkoxy, hydroxy and carboxylate. Also described is the use of a heterogeneous hydrogenation catalyst in a method of preparing a product comprising one or more product compounds selected from the group consisting of lactones having at least one group selected from carboxyl, carboalkoxy, hydroxy and carboxylate.

Lactones derived from tricarboxylic acid are an important synthetic target, because of their broad application scope on an industrial scale. Such lactones could replace monomers (e.g. for ring-opening polymerization) and solvents (e.g. gamma-butyrolactone, gamma-valerolactone) which presently are produced mainly petrochemically. Due to the finite nature and instability of fossil feedstock supply and for environmental reasons, replacement of fossil feedstock by non-fossil feedstock, i.e. feedstock obtained from renewable resources, becomes more and more important.

Radial tricarboxylic acids, e.g. citric acid, isocitric acid, tricarballylic acid and aconitic acid, esters thereof, anhydrides thereof, and salts thereof, could be used as precursors for lactones, provided that selective formation of lactones is achieved. Tricarboxylic acids like citric acid and aconitic acid are obtainable from renewable resources, e.g. from plant material by sugar fermentation.

Unfortunately, tricarboxylic acids are difficult substrates for typical reduction procedures due to the following major reasons:
- a pKA value which is incompatible with a lot of catalyst materials
- a low electrophilicity of the central C=O bond
- a strong tendency to decarboxylate with increased temperature
- a low solubility in most organic solvents
- a high reduction potential (which for example leads to neutralization with sodium hydride, but to reduction only with stronger reducing agents like lithium aluminum hydride).

Citric acid in particular is very prone to decarboxylation and dehydration at an increased temperature.

Green Chem. 2017, 19, 4642 (De Vos et al.) describes the decarboxylation and dehydration of citric acid in an aqueous system with a Ni/ZrO₂ catalyst under reductive conditions (20 bar H₂, 175 °C, 6 h) to itaconic acid as the major product (53%). The authors propose that itaconic acid then rehydrates to a β-hydroxycarboxylic acid which finally cyclizes in-situ to β-carbooyl-γ-butyrolactone. This so called "isomer hydration" products (2-(hydroxymethyl)succinic acid, β-carboxyl-γ-butyrolactone and 2-hydroxy-2-methylsuccinic acid) correspond to a fraction of 41% of the reaction products.

### Related art is also

US 4,218,381 A,
P.-F. Xu et al. / Tetrahedron Letters 46 (2005) 3815-3818,
I. Thapa et al., Catalysis Today 319 (2019) 191-196,
G. Venkateswara Rao et al., Food Chemistry 120 (2010) 235-239
H. Hida et al., Biosci., Biotechnol., Biochem. 69 (8), 1555-1561, 2005
R. Gérardy et al., Org. Process Res Dev. 2017, 21, 2012-2017
H. G. M. Walraven and U. K. Pandit, Tetrahedron Vol. 36, pp. 321 to 327.

It is a primary object of the present invention to provide a method for preparing a product comprising (a) one or more product compounds selected from the group consisting of lactones having at least one group selected from carboxyl, carboalkoxy, hydroxy and carboxylate and/or (b) one or more reaction products of one or more of said compounds selected from the group consisting of lactones having at least one group selected from carboxyl, carboalkoxy, hydroxy and carboxylate, wherein starting material obtained from renewable sources may be used.

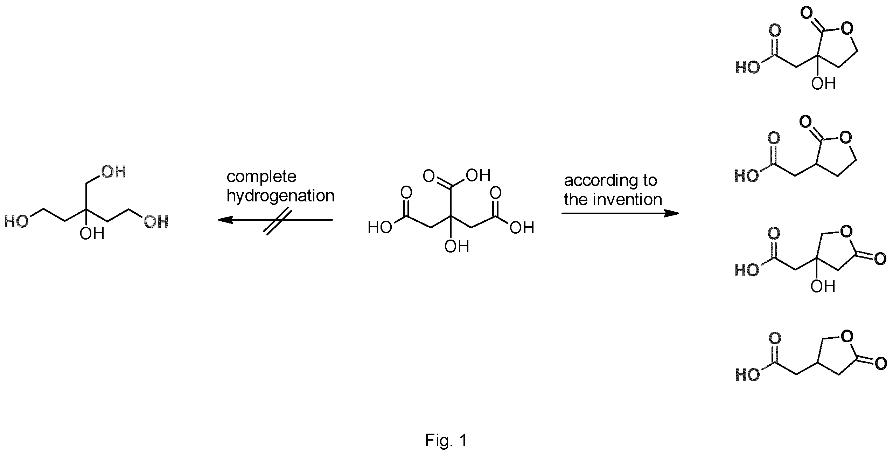

It is a further object to provide a method for selective formation of a lactone group from two carboxyl groups of an aliphatic tricarboxylic acid, two carboalkoxy groups of an ester of an aliphatic tricarboxylic acid, two carboxylate groups of a salt of an aliphatic tricarboxylic acid, or from the anhydride group of an anhydride of an aliphatic tricarboxylic acid under hydrogenation conditions, so that lactones having at least one group selected from carboxyl, carboalkoxy, hydroxy and carboxylate are obtained in a useful yield, and undesired side reactions which may result in loss of carbon atoms by decarboxylation or complete hydrogenation to polyols are suppressed (cf. fig. 1 which for the starting compound citric acid shows formation of lactones having at least one group selected from carboxyl, carboalkoxy, hydroxy and carboxylate, and the undesired complete conversion of the carboxyl groups of citric acid to hydroxy groups).

The primary object and other objects of the present invention can be accomplished by a method of preparing a product comprising
(a) one, two or more product compounds selected from the group consisting of lactones having at least one group selected from carboxyl, carboalkoxy, hydroxy and carboxylate
   and/or
(b) one or more reaction products of said one, two or more compounds selected from the group consisting of lactones having at least one group selected from carboxyl, carboalkoxy, hydroxy and carboxylate,
comprising at least the following steps:
(i) providing or preparing a starting material comprising one, two or more starting compounds selected from the group consisting of aliphatic tricarboxylic acids, esters thereof, anhydrides thereof, and salts thereof,
(ii) providing or preparing a solvent having a dielectric constant above the dielectric constant of n-butanol,
   and
(iii) chemically converting said one, two or more starting compounds provided or prepared in step (i)
   - at a temperature in the range of from 80 °C to 140 °C,
   - at a partial pressure of hydrogen in the range of from 15 MPa to 30 MPa,
   - in said solvent provided or prepared in step (ii), and
   - in the presence of a heterogeneous hydrogenation catalyst comprising one or more metals selected from the group consisting of Co, Rh, Ir, Ni, Pd and Pt,
   so that two carboxyl groups, two carboxylate groups, two carboalkoxy groups, or the anhydride group as present in said one, two or more starting compounds under hydrogenation conditions form a lactone group so that a product comprising one, two or more product compounds selected from the group consisting of lactones having at least one group selected from carboxyl, carboalkoxy, hydroxy and carboxylate results.

Surprisingly it has been found that under the above-defined hydrogenation conditions selective formation of a lactone group from two carboxyl groups of an aliphatic tricarboxylic acid, from two carboalkoxy groups of an ester of an aliphatic tricarboxylic acid, from two carboxylate groups of a salt of an aliphatic tricarboxylic acid, or from the anhydride group of an anhydride of an aliphatic tricarboxylic acid may be achieved, and product compounds selected from the group consisting of lactones having at least one group selected from carboxyl, carboalkoxy, hydroxy and carboxylate may be obtained in a useful yield, when the parameters of the hydrogenation reaction in step (iii) are selected as defined above, and a heterogeneous hydrogenation catalyst as defined above and a solvent as defined above are used. Thus, when the chemical conversion of the starting compound(s) in step (iii) is carried out using the parameters as defined above, a heterogeneous hydrogenation catalyst as defined above and a solvent as defined above, undesirable side reactions are suppressed.

Without wishing to be bound by theory, it is presently assumed that in step (iii) under hydrogenation conditions one of the carboxyl groups, carboxylate groups, carboalkoxy groups and the anhydride group of the starting compound is reduced so that a hydroxy group is formed which then undergoes a ring-closing reaction (intramolecular esterification) with another one of the carboxyl groups, carboxylate groups or carboalkoxy groups, resp. of the same molecule so that a lactone having at least one group selected from carboxyl, carboalkoxy, hydroxy and carboxylate is obtained.

When the starting compound is an aliphatic tricarboxylic acid, under hydrogenation conditions as defined above two of its carboxyl groups selectively form a lactone group, and one carboxyl group remains. When the starting compound is a salt of an aliphatic tricarboxylic acid, under hydrogenation conditions as defined above two of its carboxylate groups selectively form a lactone group, and one carboxylate group remains. When the starting compound is an ester of an aliphatic tricarboxylic acid, under hydrogenation conditions as defined above two of its carboalkoxy groups selectively form a lactone group, and one carboalkoxy group remains or is reduced to a hydroxy group under the above-defined hydrogenation conditions. When the starting compound is an anhydride of an aliphatic tricarboxylic acid, under hydrogenation conditions as defined above from the anhydride group a lactone is formed selectively, and one carboxyl group remains.

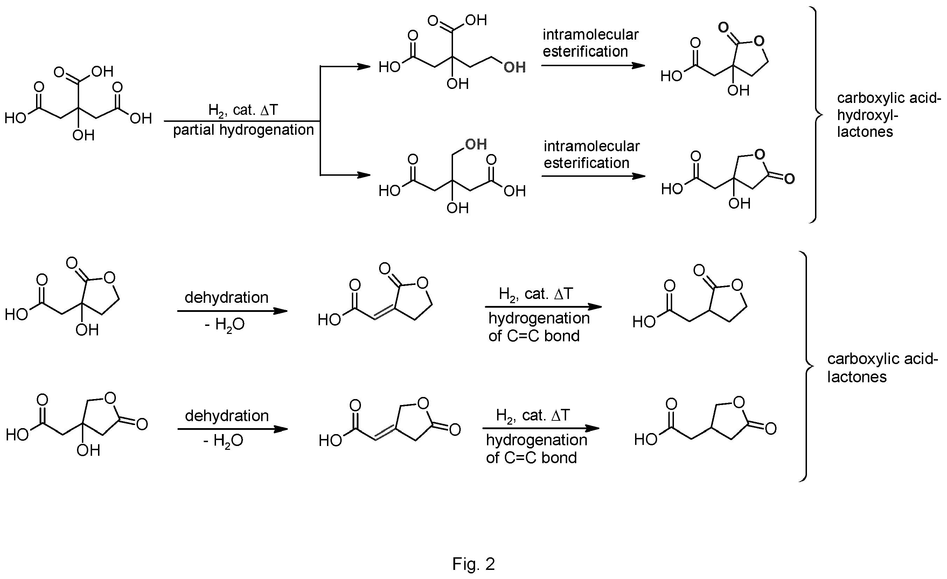

In case the starting compound is an alpha-hydroxy carboxylic acid, a salt thereof, or an anhydride thereof, the lactones formed in this way have a carboxyl group and a hydroxy group and may be dehydrated with subsequent hydrogenation to lactones having a carboxyl group and no hydroxy group (cf. fig. 2 which for the starting compound citric acid shows formation of lactones having a carboxyl group and a hydroxy group, and their subsequent dehydration and hydrogenation to lactones having a carboxyl group and no hydroxy group).

In above-defined case (a), the product resulting in step (iii), which comprises one or more product compounds selected from the group consisting of lactones having at least one group selected from carboxyl, carboalkoxy, hydroxy and carboxylate is the final product of the method. Herein, the final product comprises or consists of one, two or more product compounds selected from the group consisting of lactones having at least one group selected from carboxyl, carboalkoxy, hydroxy and carboxylate.

In above-defined case (b), the product resulting in step (iii), which comprises one or more product compounds selected from the group consisting of lactones having at least one group selected from carboxyl, carboalkoxy, hydroxy and carboxylate is an intermediate product, and said intermediate product is transferred into a final product comprising one or more reaction products of one or more of said compounds selected from the group consisting of lactones having at least one group selected from carboxyl, carboalkoxy, hydroxy and carboxylate by conducting one or more additional steps following above-defined step (iii). Herein, the intermediate product comprises or consists of one, two or more product compounds selected from the group consisting of lactones having at least one group selected from carboxyl, carboalkoxy, hydroxy and carboxylate, and the final product comprises or consists of one, two or more reaction products of one, two or more of said compounds selected from the group consisting of lactones having at least one group selected from carboxyl, carboalkoxy, hydroxy and carboxylate (for details see below).

In step (i) of the above-defined method, a starting material comprising one or more starting compounds selected from the group consisting of
- aliphatic tricarboxylic acids,
- esters of aliphatic tricarboxylic acids
- anhydrides of aliphatic tricarboxylic acids
- and salts of aliphatic tricarboxylic acids
is prepared or provided. Among these starting compounds, aliphatic tricarboxylic acids are preferred.

As understood herein, esters of aliphatic tricarboxylic acids do not include lactones.

For instance, the starting material comprises or consists of one, two or more starting compounds selected from the group consisting of aliphatic tricarboxylic acids, esters thereof, anhydrides thereof, and salts thereof.

The starting compound or at least one of said starting compounds may be selected from the group consisting of
- aliphatic tricarboxylic acids having a total number of carbon atoms of 6
- esters of aliphatic tricarboxylic acids having a total number of carbon atoms of 6
- anhydrides of aliphatic tricarboxylic acids having a total number of carbon atoms of 6
- and salts of aliphatic tricarboxylic acids having a total number of carbon atoms of 6.

Among these starting compounds, aliphatic tricarboxylic acids having a total number of carbon atoms of 6 are preferred.

For instance, the starting material comprises or consists of one, two or more starting compounds selected from the group consisting of aliphatic tricarboxylic acids having a total number of carbon atoms of 6, esters thereof, anhydrides thereof, and salts thereof. Preferably, all of the starting compounds are selected from the group consisting of aliphatic tricarboxylic acids having a total number of carbon atoms of 6, esters thereof, anhydrides thereof, and salts thereof.

In certain cases, the starting compound or at least one of said starting compounds is selected from the group consisting of
- tricarballylic acid,
- esters of tricarballylic acid,
- anhydrides of tricarballylic acid,
- and salts of tricarballylic acid.

Among these starting compounds, tricarballylic acid is preferred.

For instance, the starting material comprises or consists of one, two or more starting compounds selected from the group consisting of tricarballylic acid, esters thereof, anhydrides thereof, and salts thereof. Preferably, all of said starting compounds are selected from the group consisting of tricarballylic acid, esters thereof, anhydrides thereof, and salts thereof.

In certain cases, the starting compound or at least one of said starting compounds is selected from the group consisting of
- alpha-beta-unsaturated aliphatic tricarboxylic acids
- esters of alpha-beta-unsaturated aliphatic tricarboxylic acids
- anhydrides of alpha-beta-unsaturated aliphatic tricarboxylic acids
- and salts alpha-beta-unsaturated aliphatic tricarboxylic acids.

Among these starting compounds, alpha-beta-unsaturated aliphatic tricarboxylic acids are preferred.

For instance, the starting material comprises or consists of one, two or more starting compounds selected from the group consisting of alpha-beta-unsaturated aliphatic tricarboxylic acids, esters thereof, anhydrides thereof and salts thereof. Preferably, all of said starting compounds are selected from the group consisting of alpha-beta-unsaturated aliphatic tricarboxylic acids, esters thereof, anhydrides thereof and salts thereof.

Preferably, the alpha-beta-unsaturated aliphatic tricarboxylic acid is aconitic acid. In this case, the starting compound or at least one of said starting compounds is selected from the group consisting of
- aconitic acid,
- esters of aconitic acid
- anhydrides of aconitic acid,
- and salts of aconitic acid

Among these starting compounds, aconitic acid is preferred.

For instance, the starting material comprises or consists of one, two or more starting compounds selected from the group consisting of aconitic acid, esters thereof, anhydrides thereof, and salts thereof. Preferably, all of said starting compounds are selected from the group consisting of aconitic acid, esters thereof, anhydrides thereof, and salts thereof.

In certain cases, the starting compound or at least one of said starting compounds is selected from the group consisting of
- alpha-functionalized aliphatic tricarboxylic acids
- esters of alpha-functionalized aliphatic tricarboxylic acids
- anhydrides of alpha-functionalized aliphatic tricarboxylic acids
- and salts of alpha-functionalized aliphatic tricarboxylic acids.

Among these starting compounds, alpha-functionalized aliphatic tricarboxylic acids are preferred.

For instance, the starting material comprises or consists of one, two or more starting compounds selected from the group consisting of alpha-functionalized aliphatic tricarboxylic acids, esters thereof, anhydrides thereof and salts thereof. Preferably, all of said starting compounds are selected from the group consisting of alpha-functionalized aliphatic tricarboxylic acids, esters thereof, anhydrides thereof and salts thereof.

Preferably, the alpha-functionalized aliphatic tricarboxylic acids are alpha-hydroxy aliphatic carboxylic acids. In this case, the starting compound or at least one of said starting compounds is selected from the group consisting of
- alpha-hydroxy aliphatic tricarboxylic acids,
- esters of alpha-hydroxy aliphatic tricarboxylic acids
- anhydrides of alpha-hydroxy aliphatic tricarboxylic acids
- and salts of alpha-hydroxy aliphatic tricarboxylic acids.

Among these starting compounds, alpha-hydroxy aliphatic tricarboxylic acids are preferred.

For instance, the starting material comprises or consists of one, two or more starting compounds selected from the group consisting of alpha-hydroxy aliphatic tricarboxylic acids, esters thereof, anhydrides thereof and salts thereof. Preferably, all of said starting compounds are selected from the group consisting of alpha-hydroxy aliphatic tricarboxylic acids, esters thereof, anhydrides thereof and salts thereof.

Preferred alpha-hydroxy aliphatic tricarboxylic acids are citric acid and isocitric acid. In this case, the starting compound or at least one of said starting compounds is selected from the group consisting of
- citric acid and isocitric acid,
- esters of citric acid and esters of isocitric acid
- anhydrides of citric acid and anhydrides of isocitric acid
- salts of citric acid and salts of isocitric acid.

Among these starting compounds, citric acid and isocitric acid are preferred.

For instance, the starting material comprises or consists of one, two or more starting compounds selected from the group consisting of citric acid, isocitric acid, esters thereof, anhydrides thereof and salts thereof. Preferably, all of said starting compounds are selected from the group consisting of citric acid, isocitric acid, esters thereof, anhydrides thereof and salts thereof.

The most preferred starting compounds in the group consisting of alpha-hydroxy aliphatic tricarboxylic acids, esters thereof, anhydrides thereof and salts thereof are citric acid, triethyl citrate, and isocitric acid. For instance, the starting material comprises or consists of one, two or all of citric acid, triethyl citrate, and isocitric acid. Preferably, all of said starting compounds are selected from the group consisting of citric acid, triethyl citrate, and isocitric acid.

The above mentioned specific preferred starting compounds are aliphatic tricarboxylic acids having a structure according to formula (I), esters thereof, anhydrides thereof, and salts thereof 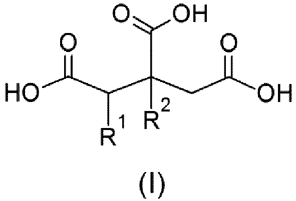 wherein
(i) R¹ = H; R² = H (tricarballylic acid), or
(ii) R¹ = OH; R² = H (isocitric acid) or
(iii) R¹ = H; R² = OH (citric acid) or
(iv) R', R² together designate a double bond (aconitic acid).

Thus, the starting compound or at least one of said starting compounds may be selected from the group consisting of
- citric acid,
- isocitric acid,
- aconitic acid,
- tricarballylic acid,
- esters of acids selected from the group consisting of citric acid, isocitric acid, aconitic acid and tricarballylic acid,
- anhydrides of acids selected from the group consisting of citric acid, isocitric acid, aconitic acid and tricarballylic acid,
- and salts of acids selected from the group consisting of citric acid, isocitric acid, aconitic acid and tricarballylic acid.

Among these starting compounds, citric acid, isocitric acid, aconitic acid and tricarballylic acid are preferred.

For instance, the starting material comprises or consists of one, two or more starting compounds selected from the group consisting of citric acid, isocitric acid, aconitic acid, tricarballylic acid, esters thereof, anhydrides thereof, and salts thereof. Preferably, all of said starting compounds are selected from the group consisting of citric acid, isocitric acid, aconitic acid, tricarballylic acid, esters thereof, anhydrides thereof, and salts thereof.

A specially preferred starting compound is citric acid. For instance, the starting material comprises or consists of citric acid. Preferably, the starting material consists of citric acid.

The total concentration of starting compounds selected from the group consisting of aliphatic tricarboxylic acids, esters thereof, anhydrides thereof, and salts thereof may be 5 wt.-% or more, preferably 20 wt.-% or more, based on the total amount of the reaction mixture at the beginning of step (iii). The total concentration of all starting compounds selected from the group consisting of aliphatic tricarboxylic acids, esters thereof, anhydrides thereof, and salts thereof may be 60 wt.-% or less, preferably 55 wt.-% or less, based on the total amount of the reaction mixture at the beginning of step (iii).

Herein, the total amount of the reaction mixture at the beginning of step (iii) is the sum of the amount of the starting compounds and the solvent having a having a dielectric constant above the dielectric constant of n-butanol.

Preferably, the total concentration of starting compounds selected from the group consisting of aliphatic tricarboxylic acids, esters thereof, anhydrides thereof, and salts thereof is 5 wt.-% or more and 60 wt.-% or less, based on the total amount of the reaction mixture at the beginning of step (iii). More preferably, the total concentration of starting compounds selected from the group consisting of aliphatic tricarboxylic acids, esters thereof, anhydrides thereof, and salts thereof is 20 wt.-% or more and 55 wt.-% or less, based on the total amount of the reaction mixture at the beginning of step (iii).

The starting material comprising one, two or more starting compounds selected from the group consisting of aliphatic tricarboxylic acids, esters thereof, anhydrides thereof, and salts thereof, preferably selected from the group consisting of citric acid and aconitic acid, is preferably prepared or isolated from plant material. Thus, the above-defined method has the advantage that starting material obtained from renewable sources may be used.

For instance, the starting material is prepared from plant material by sugar fermentation.

Another potential renewable source for starting materials, especially citric acid and starting compounds obtainable from chemical conversion of citric acid, are citrus fruits.

The product resulting in step (iii) of the above-defined method comprises one or more product compounds selected from the group consisting of lactones having at least one group selected from carboxyl, carboalkoxy, hydroxy and carboxylate. For instance, the product resulting in step (iii) may comprise or consist of one, two or more product compounds selected from the group consisting of lactones having one carboxyl group and one hydroxy group and lactones having one carboxyl group and no hydroxy group.

Preferably, all product compounds are compounds selected from the group consisting of lactones having one carboxyl group and one hydroxy group and lactones having one carboxyl group and no hydroxy group. Preferred product compounds selected from the group consisting of lactones having one carboxyl group and one hydroxy group are tetrahydro-3-hydroxy-5-oxo-3-furanacetic acid and tetrahydro-3-hydroxy-2-oxo-3-furanacetic acid. Preferred product compounds selected from the group consisting of lactones having one carboxyl group and no hydroxy group are tetrahydro-5-oxo-3-furanacetic acid and tetrahydro-2-oxo-3-furanacetic acid.

In certain preferred cases the product resulting in step (iii) comprises two or more product compounds selected from the group consisting of tetrahydro-3-hydroxy-5-oxo-3-furanacetic acid, tetrahydro-3-hydroxy-2-oxo-3-furanacetic acid, tetrahydro-5-oxo-3-furanacetic acid, and tetrahydro-2-oxo-3-furanacetic acid.

Tetrahydro-3-hydroxy-5-oxo-3-furanacetic acid has CAS No. 98136-18-6.

Tetrahydro-3-hydroxy-2-oxo-3-furanacetic acid has not been described so far and accordingly has no CAS No.

Tetrahydro-5-oxo-3-furanacetic acid has CAS No. 5807-39-6.

Tetrahydro-2-oxo-3-furanacetic acid has CAS No. 13281-16-8.

From starting compounds selected from the group consisting of citric acid (exemplarily shown below), esters thereof, salts thereof and anhydrides thereof, the product compounds tetrahydro-3-hydroxy-5-oxo-3-furanacetic acid, tetrahydro-3-hydroxy-2-oxo-3-furanacetic acid, tetrahydro-5-oxo-3-furanacetic acid, and tetrahydro-2-oxo-3-furanacetic acid may be obtained by the method according to the present invention:

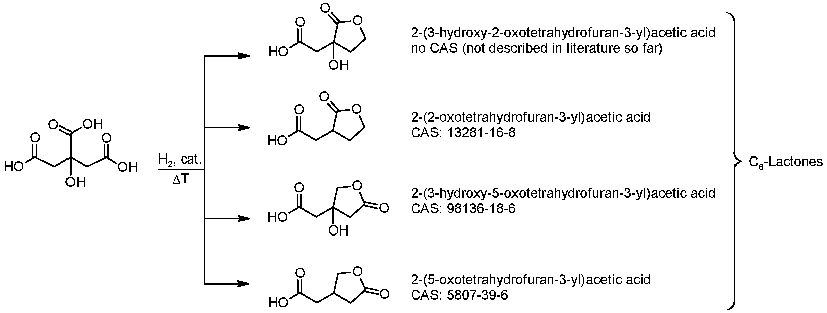

From starting compounds selected from the group consisting of aconitic acid (exemplarily shown below), esters thereof, salts thereof and anhydrides thereof, the product compounds tetrahydro-5-oxo-3-furanacetic acid, and tetrahydro-2-oxo-3-furanacetic acid may be obtained by the method according to the present invention:

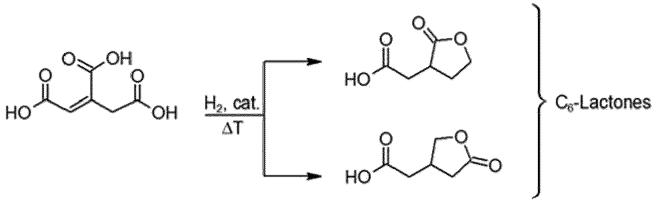

From starting compounds selected from the group consisting of tricarballylic acid, esters thereof, salts thereof and anhydrides thereof, the product compounds tetrahydro-5-oxo-3-furanacetic acid and tetrahydro-2-oxo-3-furanacetic acid may be obtained by the method according to the present invention.

From starting compounds selected from the group consisting of isocitric acid (exemplarily shown below), esters thereof, salts thereof and anhydrides thereof, the following product compounds may be obtained by the method according to the present invention:

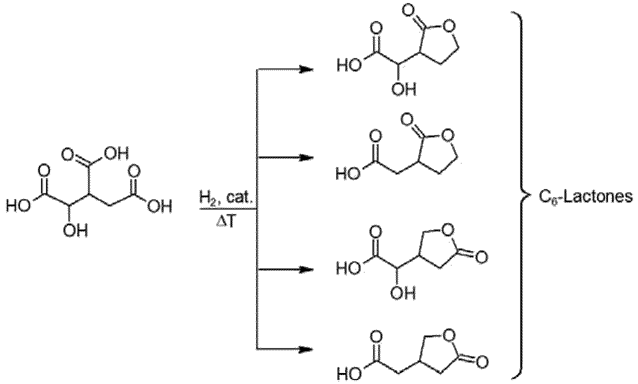

In the product resulting in step (iii) the total amount of lactones having at least one group selected from carboxyl, carboalkoxy, hydroxy and carboxylate may be 50 wt.-% or more, preferably 60 wt.-% or more, or in the range of from 50 wt.% to 95 wt.-%, in each case based on the total amount of residual starting compounds and product compounds obtained by chemical conversion of the one or more starting compounds, including product compounds which are not lactones having at least one group selected from carboxyl, carboalkoxy, hydroxy and carboxylate. It is understood that a certain amount of product compounds which are not lactones having at least one group selected from carboxyl, carboalkoxy, hydroxy and carboxylate may result from unavoidable side reactions.

Preferably, in the product resulting in step (iii) the total amount of product compounds selected from the group consisting of tetrahydro-3-hydroxy-5-oxo-3-furanacetic acid, tetrahydro-3-hydroxy-2-oxo-3-furanacetic acid, tetrahydro-5-oxo-3-furanacetic acid, and tetrahydro-2-oxo-3-furanacetic acid, is 50 wt.-% or more, preferably 60 wt.-% or more, or in the range of from 50 wt.% to 95 wt.-%, in each case based on the total amount of residual starting compounds and product compounds obtained by chemical conversion of the one or more starting compounds, including product compounds which are not lactones having at least one group selected from carboxyl, carboalkoxy, hydroxy and carboxylate. It is understood that a certain amount of product compounds which are not lactones having at least one group selected from carboxyl, carboalkoxy, hydroxy and carboxylate may result from unavoidable side reactions.

When an aliphatic tricarboxylic acid or a salt thereof or the anhydride thereof is used as a starting compound, it is preferably chemically converted in step (iii) so that predominantly the number of carbon atoms present in said starting compound is maintained so that the resulting lactone(s) has/have the same number of carbon atoms as said starting compound. Thus, predominantly no carbon atom of the starting compound is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When an aliphatic tricarboxylic acid having a number of carbon atoms of 6 or a salt thereof or the anhydride thereof is used as a starting compound, it is preferably chemically converted in step (iii) so that predominantly the number of carbon atoms present in said starting compound is maintained so that the resulting lactone(s) has/have 6 carbon atoms including a carboxyl group. Thus, predominantly no carbon atom of the starting compound is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having 6 carbon atoms, residual starting compounds, and product compounds formed by side reactions which are not lactones having 6 carbon atoms.

When an ester of an aliphatic tricarboxylic acid is used as a starting compound, it is preferably chemically converted in step (iii) so that predominantly the number of carbon atoms present in the aliphatic tricarboxylic acid corresponding to said ester is maintained so that the resulting lactone(s) either has/have the a number of carbon atoms which is the sum of the number of carbon atoms in the aliphatic tricarboxylic acid corresponding to the ester used as the starting compound and the number of carbon atoms in the remaining carboalkoxy group (which is not involved in formation of the lactone group) of the starting compound, or in case the carboalkoxy group of the starting compound which is not involved in formation of the lactone group is reduced to a hydroxy group under the above-defined hydrogenation conditions, the resulting lactone(s) has/have the same number of carbon atoms as said aliphatic tricarboxylic acid corresponding to the ester used as the starting compound. Thus, predominantly no carbon atom of the aliphatic tricarboxylic acid corresponding to said ester used as the starting compound is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When an ester of an aliphatic tricarboxylic acid having a number of carbon atoms of 6 is used as a starting compound, it is preferably chemically converted in step (iii) so that predominantly the number of carbon atoms present in the aliphatic tricarboxylic acid corresponding to said ester is maintained so that the resulting lactone(s) either has/have a number of carbon atoms which is the sum of 6 and the number of carbon atoms in the remaining carboalkoxy group (which is not involved in formation of the lactone group) of the starting compound, or in case the carboalkoxy group not involved in formation of the lactone group is reduced to a hydroxy group under the above-defined hydrogenation conditions, the resulting lactone(s) has/have 6 carbon atoms. Thus, predominantly no carbon atom of the aliphatic tricarboxylic acid corresponding to the ester used as the starting compound is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having 6 carbon atoms including a carboxyl group, residual starting compounds, and product compounds formed by side reactions which are not lactones having at least one group selected from carboxyl, carboalkoxy, hydroxy and carboxylate.

When an aliphatic tricarboxylic acid or a salt thereof or the anhydride thereof is used as a starting compound, it is preferably chemically converted in step (iii) so that predominantly the number of carbon-carbon single bonds present in said starting compound is maintained so that the resulting lactone(s) has/have the same number of carbon-carbon single bonds as said starting compound. Thus, predominantly no carbon-carbon single bond of the starting compound is converted into a carbon-carbon double bond by dehydratation, or in case a carbon-carbon double bond is formed intermediary, it is hydrogenated in-situ (cf. fig. 2). Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When an ester of an aliphatic tricarboxylic acid is used as a starting compound, it is preferably chemically converted in step (iii) so that predominantly the number of carbon-carbon single bonds present in the aliphatic tricarboxylic acid corresponding to said ester is maintained so that the resulting lactone(s) has/have a number of carbon-carbon single bonds which is the sum of the number of carbon-carbon single bonds in the aliphatic tricarboxylic acid corresponding to the ester used as the starting compound and the number of carbon-carbon single bonds in the remaining carboalkoxy group (which is not involved in formation of the lactone group) of the starting compound, or in case the carboalkoxy group of the starting compound which is not involved in formation of the lactone group is reduced to a hydroxy group under the above-defined hydrogenation conditions, the resulting lactone(s) has/have the same number of carbon-carbon single bonds as said aliphatic tricarboxylic acid corresponding to the ester used as the starting compound. Thus, predominantly no carbon-carbon single bond of the aliphatic tricarboxylic acid corresponding to said ester used as the starting compound is converted into a carbon-carbon double bond by dehydratation, or in case a carbon-carbon double bond is formed intermediary, it is hydrogenated in-situ. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When an alpha-beta unsaturated aliphatic tricarboxylic acid or a salt thereof or an anhydride thereof is used as a starting compound, it is preferably chemically converted in step (iii) so that predominantly the carbon-carbon double bond present in said starting compound is converted into a carbon-carbon single bond so that the resulting lactone(s) has/have one carbon-carbon single bond more than said starting compound. Thus, predominantly the carbon-carbon double bond of the starting compound is hydrogenated in-situ. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When an ester of an alpha-beta unsaturated aliphatic tricarboxylic acid is used as a starting compound, it is preferably chemically converted in step (iii) so that predominantly the carbon-carbon double bond present in the aliphatic tricarboxylic acid corresponding to said ester is converted into a carbon-carbon single bond so that the resulting lactone(s) either has/have one carbon-carbon-single bond more than the sum of the number of carbon-carbon single bonds in the alpha-beta unsaturated aliphatic tricarboxylic acid corresponding to the ester used as the starting compound and the number of carbon-carbon single bonds in the remaining carboalkoxy group (which is not involved in formation of the lactone group) of the starting compound, or in case the carboalkoxy group of the starting compound which is not involved in formation of the lactone group is reduced to a hydroxy group under the above-defined hydrogenation conditions, the resulting lactone(s) has/have one carbon-carbon single bond more than said alpha-beta unsaturated aliphatic tricarboxylic acid corresponding to the ester used as the starting compound. Thus, predominantly the carbon-carbon double bond of the alpha-beta unsaturated aliphatic tricarboxylic acid corresponding to the ester used as the starting compound is hydrogenated in-situ. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds and product compounds formed by side reactions which are not lactones having at least one group selected from carboxyl, carboalkoxy, hydroxy and carboxylate.

When citric acid or a salt thereof or the anhydride thereof is used as a starting compound, it is preferably chemically converted in step (iii) so that predominantly the number of carbon atoms present in citric acid is maintained so that the resulting lactone(s) has/have the same number of carbon atoms as citric acid. Thus, predominantly no carbon atom of citric acid is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When an ester of citric acid is used as a starting compound, it is preferably chemically converted in step (iii) so that predominantly the number of carbon atoms present in citric acid is maintained so that the resulting lactone(s) either has/have a number of carbon atoms which is the sum of 6 and the number of carbon atoms in the remaining carboalkoxy group (which is not involved in formation of the lactone group) of said ester of citric acid, or in case the carboalkoxy group of said ester of citric acid not involved in formation of the lactone group is reduced to a hydroxy group under the above-defined hydrogenation conditions, the resulting lactone(s) has/have the same number of carbon atoms as citric acid. Thus, predominantly no carbon atom of citric acid is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When citric acid or a salt thereof or the anhydride thereof is used as a starting compound, it is preferably chemically converted in step (iii) so that predominantly the number of carbon-carbon single bonds present in citric acid is maintained so that the resulting lactone(s) has/have the same number of carbon-carbon single bonds as citric acid. Thus, predominantly no carbon-carbon single bond of citric acid is converted into a carbon-carbon double bond by dehydratation, or in case a carbon-carbon double bond is formed intermediary, it is hydrogenated in-situ (cf. fig. 2). Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When an ester of citric acid is used as a starting compound, it is preferably chemically converted in step (iii) so that predominantly the number of carbon-carbon single bonds present in citric acid is maintained so that the resulting lactone(s) has/have a number of carbon-carbon single bonds corresponding to the sum of the number of carbon-carbon single bonds in citric acid and the number of carbon-carbon single bonds in the remaining carboalkoxy group (which is not involved in formation of the lactone group) of said ester of citric acid, or in case the carboalkoxy group of said ester of citric acid not involved in formation of the lactone group is reduced to a hydroxy group under the above-defined hydrogenation conditions, the resulting lactone(s) has/have the same number of carbon-carbon single bonds as citric acid. Thus, predominantly no carbon-carbon single bond of citric acid is converted into a carbon-carbon double bond by dehydratation, or in case a carbon-carbon double bond is formed intermediary, it is hydrogenated in-situ. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

In step (ii) of the above-defined method, a solvent having a dielectric constant above the dielectric constant of n-butanol is prepared or provided. Thus, the solvent has a higher polarity than n-butanol. Preferred solvents are protic. Such solvents have a sufficient solubility for the above-defined starting compounds.

The solvent provided or prepared in step (ii) may comprise one or more constituents selected from the group consisting of water, methanol, ethanol, n-propanol, iso-propanol, ethylene glycols, propylene glycols and cyclic ethers in such proportions that a dielectric constant above the dielectric constant of n-butanol results. Typical cyclic ethers are tetrahydrofurane (THF), tetrahydropyrane (THP), and dioxanes.

Preferably the solvent is selected from the group consisting of
- water,
   and
- aqueous mixtures comprising water in an amount of more than 50 wt.-%, preferably more than 70 wt.-%, more preferably more than 90 wt.-%, based on the total amount of the solvent.

In said aqueous mixtures, one or more of methanol, ethanol, n-propanol, iso-propanol, ethylene glycols, propylene glycols, cyclic ethers may be admixed to water.

In step (iii) of the above-defined method, said one or more starting compounds provided or prepared in step (i) may be chemically converted into one or more of the above-defined product compounds at a temperature in the range of from 80 °C to 125 °C, preferably at temperatures in the range of from 95 °C to 120 °C.

Depending on the reaction temperature, chemical conversion of starting compounds selected from the group consisting of aliphatic tricarboxylic acids, esters thereof, anhydrides thereof, and salts thereof at a partial pressure of hydrogen in the range of from 15 MPa to 30 MPa in the presence of a given heterogeneous hydrogenation catalyst may result either in aliphatic polyols (not according to the invention, cf. fig. 1) or lactones having at least one group selected from carboxyl, carboalkoxy, hydroxy and carboxylate, or mixtures of both kinds of products. Therefore, in step (iii), the reaction temperature is preferably chosen so as to selectively prepare said lactones having at least one group selected from carboxyl, carboalkoxy, hydroxy and carboxylate.

Step (iii) of the above-defined method may be conducted for a period of 10 hours or more, preferably 20 hours or more. Step (iii) of the above-defined method may be conducted for a period of 200 hours or less, preferably 140 hours or less, more preferably 72 hours or less. Preferably, step (iii) of the above-defined method is conducted for a period of 10 hours or more, and 200 hours or less. More preferably, step (iii) of the above-defined method is conducted for a period of 20 hours or more, and 140 hours or less, preferably 72 hours or less.

In step (iii) of the above-defined method, said one or more starting compounds provided or prepared in step (i) are chemically converted into one or more of the above-defined product compounds in the presence of a heterogeneous hydrogenation catalyst comprising one or more metals selected from the group consisting of Co, Rh, Ir, Ni, Pd and Pt.

Preferred heterogeneous hydrogenation catalysts comprise or consist of one or more metals selected from the group consisting of Co, Rh, Ir, Ni, Pd and Pt, in a total amount of 90 wt.-% or more, preferably 95 wt.-% or more, based on the total amount of the heterogeneous hydrogenation catalyst. Preferably the total amount of Pd and Rh is 90 wt.-% or more, preferably 95 wt.-% or more. Further preferably the amount of either Pd or Rh is 90 wt.-% or more, preferably 95 wt.-% or more, based on the total amount of the heterogeneous hydrogenation catalyst.

It is understood that in each case the catalyst may comprise traces of other metals and of oxides, which are included in the above-mentioned amount of the heterogeneous hydrogenation catalyst.

Preferably, the amount of heterogeneous hydrogenation catalyst relative to the total amount of the starting compounds is in the range of from 0.05 wt% to 10 wt%, preferably 0.1 wt% to 7.5 wt%.

The heterogeneous hydrogenation catalyst may be supported by a support material (also referred to as a carrier). The weight of the support material is not included in the above-mentioned amount of the heterogeneous hydrogenation catalyst.

The support material of the heterogeneous hydrogenation catalyst is preferably chosen so as to sustain the hydrothermal stress resulting from the simultaneous presence of heat, water, acid and hydrogenolytic conditions during step (iii) of the above-defined method.

Preferably, the support material is selected from the group consisting of metal oxides, zeolites and carbon-based materials, preferably Al₂O₃, ZrO₂, TiO₂, SiC, carbon black and PTFE. Combinations of different support materials are possible, e.g. PTFE-supported carbon black

In supported catalysts, preferably the total amount of heterogeneous hydrogenation catalyst is in the range of from 1 wt.-% to 15 wt.-%, more preferably 5 wt.-% to 10 wt.-%, relative to the sum of the weight of the heterogeneous hydrogenation catalyst and the support material.

Preferred combinations of catalyst metals and support materials are Pd supported on carbon black and Rh supported on Al₂O₃.

After step (iii), the supported heterogeneous hydrogenation catalyst may be recovered, preferably be means of filtration.

The above-defined method may comprise one or more additional steps which are conducted after above-defined step (iii).

In an additional step conducted after above-defined step (iii), the solvent used in step (iii) may be removed by evaporation. Evaporation of the solvent may be carried out by any suitable method. For instance, evaporation of the solvent may be carried out by freeze-drying.

In an additional step conducted after above-defined step (iii), one or more of said product compounds selected from the group consisting of lactones having at least one group selected from carboxyl, carboalkoxy, hydroxy and carboxylate present in the product resulting from step (iii) may be chemically converted to give a product (b) comprising one or more reaction products of one, two or more of said compounds selected from the group consisting of lactones having at least one group selected from carboxyl, carboalkoxy, hydroxy and carboxylate. Herein, the product resulting in step (iii), which comprises one or more product compounds selected from the group consisting of lactones having at least one group selected from carboxyl, carboalkoxy, hydroxy and carboxylate is an intermediate product, and said intermediate product is chemically converted into a final product comprising one or more reaction products of one or more of said compounds selected from the group consisting of lactones having at least one group selected from carboxyl, carboalkoxy, hydroxy and carboxylate.

In an additional step conducted after above-defined step (iii), one or more additional chemical substances may be added to one or more of said product compounds selected from the group consisting of lactones having at least one group selected from carboxyl, carboalkoxy, hydroxy and carboxylate present in the product resulting from step (iii) so that a reaction mixture results comprising said one or more product compounds selected from the group consisting of lactones having at least one group selected from carboxyl, carboalkoxy, hydroxy and carboxylate. Thus, a reaction mixture is formed which contains
- one, two or more lactones having at least one group selected from carboxyl, carboalkoxy, hydroxy and carboxylate
- one or more additional chemical substances which are not lactones having at least one group selected from carboxyl, carboalkoxy, hydroxy and carboxylate.

Such reaction mixtures are configured and intended for being used for preparing reaction products (for details see below) different from the above-mentioned product comprising one or more product compounds selected from the group consisting of lactones having at least one group selected from carboxyl, carboalkoxy, hydroxy and carboxylate, which results from above-defined step (iii).

In said reaction mixture, said one, two or more product compounds selected from the group consisting of lactones having at least one group selected from carboxyl, carboalkoxy, hydroxy and carboxylate may function as a reactant or as a solvent.

The above-defined method may comprise one, two or all of the above-defined additional steps which are conducted after above-defined step (iii).

As explained above, in case (a) the product of the above-defined method comprises one or more product compounds selected from the group consisting of lactones having at least one group selected from carboxyl, carboalkoxy, hydroxy and carboxylate.

Product (a) may be selected from the group consisting of formulations comprising one or more materials selected from the group consisting of:
- peptides,
- proteins, wherein preferably the proteins are selected from the group consisting of human serum albumin and bovine serum albumin,
- enzymes, wherein preferably the enzymes are selected from the group consisting of lysozymes, proteases, amylases, lipases, proteases, mannanases, and cellulases,
- microorganisms, wherein preferably the microorganisms are selected from the group consisting of gram-positive bacteria, gram-negative bacteria, spore forming bacteria, fungal spore, mycelia, yeasts,
- DNA, RNA and viruses, wherein preferably the viruses are selected from the group consisting of bacteriophages.

Formulations comprising one or more materials selected from the group consisting of peptides, proteins, enzymes, DNA, RNA, viruses and microorganisms are used for a wide variety of different applications including biocatalysis, food sector, feed applications, home care, personal care and agriculture.

In such formulations the product compounds selected from the group consisting of lactones having at least one group selected from carboxyl, carboalkoxy, hydroxy and carboxylate may substitute conventional solvents like 1,2-propanediol, glycerol or sorbitol, and/or may protect and stabilize the materials selected from the group consisting of peptides, proteins, enzymes, DNA, RNA, viruses and microorganisms. More specifically, the product compounds selected from the group consisting of lactones having at least one group selected from carboxyl, carboalkoxy, hydroxy and carboxylate may be used as a biostatic agent to avoid proliferation of microorganisms.

Product (a) may be a mixture comprising metal cations, preferably Fe, Mg, Ca, Sr, Cu, Ag and Au cations, complexed by said one, two or more product compounds selected from the group consisting of lactones having at least one group selected from carboxyl, carboalkoxy, hydroxy and carboxylate, wherein the mixture is prepared after step (iii) of chemically converting said one or more starting compounds. Such mixture may be selected from the group consisting of refinery products, mining products, and home care products.

Product (a) may be a reaction mixture for preparing polymers, preferably for preparing polymers by ring-opening polymerization or by alkoxylation with ethylene oxide and/or propylene oxide, wherein the reaction mixture is prepared after step (iii) of chemically converting said one or more starting compounds.

By ring-opening polymerization, polymers from the group of polyesters are obtainable.

Product (a) may be a reaction mixture for preparing amides or esters of said one, two or more product compounds selected from the group consisting of lactones having at least one group selected from carboxyl, carboalkoxy, hydroxy and carboxylate, wherein the reaction mixture is prepared after step (iii) of chemically converting said one or more starting compounds. Preferred esters are methyl esters and ethyl esters. Said esters may be used as aroma components. Preferred amides are methyl amides and ethyl amides.

The above-mentioned reaction mixtures may be obtained by adding one or more additional chemical substances to one, two or more of said product compounds selected from the group consisting of lactones having at least one group selected from carboxyl, carboalkoxy, hydroxy and carboxylate in an additional step conducted after step (iii) of chemically converting said one or more starting compounds.

As explained above, in case (b) the product of the above-defined method comprises one or more reaction products of one or more of said compounds selected from the group consisting of lactones having at least one group selected from carboxyl, carboalkoxy, hydroxy and carboxylate.

Product (b) may be selected from the group consisting of products comprising one or more polymers, preferably polyalkoxylates or polyesters, wherein at least one of said polymers is prepared from said one, two or more product compounds selected from the group consisting of lactones having at least one group selected from carboxyl, carboalkoxy, hydroxy and carboxylate in an additional step conducted after step (iii) of chemically converting said one or more starting compounds.

Product (b) may be selected from the group consisting of solutions comprising as a solvent or solvent constituent one or more reaction products of said one, two or more product compounds selected from the group consisting of lactones having at least one group selected from carboxyl, carboalkoxy, hydroxy and carboxylate, wherein preferably said one or more reaction products are selected from the group of amides and esters of said one, two or more product compounds selected from the group consisting of lactones having at least one group selected from carboxyl, carboalkoxy, hydroxy and carboxylate.

In a specifically preferred method according to the present invention, a product comprising
(a) one, two, three or all product compounds selected from the group consisting of tetrahydro-3-hydroxy-5-oxo-3-furanacetic acid, tetrahydro-3-hydroxy-2-oxo-3-furanacetic acid, tetrahydro-5-oxo-3-furanacetic acid and tetrahydro-2-oxo-3-furanacetic acid
   and/or
(b) one or more reaction products of one, two, three or all of said product compounds is prepared by a method comprising at least the following steps:
   (i) providing or preparing a starting material comprising one, two or more starting compounds selected from the group consisting of citric acid, esters thereof, anhydrides thereof, and salts thereof,
   (ii) providing or preparing a solvent having a dielectric constant above the dielectric constant of n-butanol, wherein said solvent is water or a mixture of water with one selected from the group consisting of methanol and ethylene glycols,
      and
   (iii) chemically converting said one, two or more starting compounds provided or prepared in step (i)
      - at a temperature in the range of from 80 °C to 125 °C,
      - at a partial pressure of hydrogen in the range of from 15 MPa to 30 MPa,
      - in said solvent provided or prepared in step (ii), and
      - in the presence of a heterogeneous hydrogenation catalyst comprising Pd or Rh,
      so that two carboxyl groups, two carboxylate groups, two carboalkoxy groups, or the anhydride group as present in said one, two or more starting compounds under hydrogenation conditions form a lactone group so that a product comprising one, two or more product compounds selected from the group consisting of tetrahydro-3-hydroxy-5-oxo-3-furanacetic acid, tetrahydro-3-hydroxy-2-oxo-3-furanacetic acid, tetrahydro-5-oxo-3-furanacetic acid and tetrahydro-2-oxo-3-furanacetic acid results.

In another specifically preferred method according to the present invention, a product comprising
(a) one or both product compounds selected from the group consisting of tetrahydro-5-oxo-3-furanacetic acid and tetrahydro-2-oxo-3-furanacetic acid
   and/or
(b) one or more reaction products of one or both of said product compounds is prepared by a method comprising at least the following steps:
   (i) providing or preparing a starting material comprising one, two or more starting compounds selected from the group consisting of aconitic acid, esters thereof, anhydrides thereof, and salts thereof,
   (ii) providing or preparing a solvent having a dielectric constant above the dielectric constant of n-butanol, wherein said solvent is water or a mixture of water with one selected from the group consisting of methanol and ethylene glycols,
      and
   (iii) chemically converting said one, two or more starting compounds provided or prepared in step (i)
      - at a temperature in the range of from 80 °C to 125 °C,
      - at a partial pressure of hydrogen in the range of from 15 MPa to 30 MPa,
      - in said solvent provided or prepared in step (ii), and
      - in the presence of a heterogeneous hydrogenation catalyst comprising Pd or Rh,
      so that two carboxyl groups, two carboxylate groups, two carboalkoxy groups, or the anhydride group as present in said one, two or more starting compounds under hydrogenation conditions form a lactone group so that a product comprising one or both product compounds selected from the group consisting of tetrahydro-5-oxo-3-furanacetic acid and tetrahydro-2-oxo-3-furanacetic acid results.

In a further specifically preferred method according to the present invention, a product comprising
(a) one or both product compounds selected from the group consisting of tetrahydro-5-oxo-3-furanacetic acid and tetrahydro-2-oxo-3-furanacetic acid
   and/or
(b) one or more reaction products of one or both of said product compounds is prepared by a method comprising at least the following steps:
   (i) providing or preparing a starting material comprising one, two or more starting compounds selected from the group consisting of tricarballylic acid, esters thereof, anhydrides thereof, and salts thereof,
   (ii) providing or preparing a solvent having a dielectric constant above the dielectric constant of n-butanol, wherein said solvent is water or a mixture of water with one selected from the group consisting of methanol and ethylene glycols,
      and
   (iii) chemically converting said one, two or more starting compounds provided or prepared in step (i)
      - at a temperature in the range of from 80 °C to 125 °C,
      - at a partial pressure of hydrogen in the range of from 15 MPa to 30 MPa,
      - in said solvent provided or prepared in step (ii), and
      - in the presence of a heterogeneous hydrogenation catalyst comprising Pd or Rh,
      so that two carboxyl groups, two carboxylate groups, two carboalkoxy groups, or the anhydride group as present in said one, two or more starting compounds under hydrogenation conditions form a lactone group so that a product comprising one or both product compounds selected from the group consisting of tetrahydro-5-oxo-3-furanacetic acid and tetrahydro-2-oxo-3-furanacetic acid results.

The present application also relates to a product selected from the group consisting of
- formulations comprising one or more materials selected from the group consisting of:
   - peptides,
   - proteins, wherein preferably the proteins are selected from the group consisting of human serum albumin and bovine serum albumin,
   - enzymes, wherein preferably the enzymes are selected from the group consisting of lysozymes, proteases, amylases, lipases, proteases, mannanases, and cellulases,
   - microorganisms, wherein preferably the microorganisms are selected from the group consisting of gram-positive bacteria, gram-negative bacteria, spore forming bacteria, fungal spore, mycelia, yeasts,
   - DNA, RNA and viruses, wherein preferably the viruses selected from the group consisting of bacteriophages
- refinery products comprising complexing agents,
- mining products comprising complexing agents,
- home care products comprising complexing agents,
- reaction mixtures for converting said one, two or more product compounds selected from the group consisting of lactones having at least one group selected from carboxyl, carboalkoxy, hydroxy and carboxylate into a corresponding polymer, preferably by ring-opening polymerization or by alkoxylation with ethylene oxide and/or propylene oxide, wherein the reaction mixture is prepared after step (iii), and
- reaction mixtures for preparing esters or amides, more preferably methyl esters, ethyl esters, methyl amides or ethyl amides, of said one, two or more product compounds selected from the group consisting of lactones having at least one group selected from carboxyl, carboalkoxy, hydroxy and carboxylate, wherein the reaction mixture is prepared after step (iii)
wherein the product comprises one, two or more product compounds selected from the group consisting of lactones having at least one group selected from carboxyl, carboalkoxy, hydroxy and carboxylate.

Preferably, an above-defined product comprises lactones selected from the group consisting of
- lactones having one carboxyl group and one hydroxy group, and
- lactones having one carboxyl group and no hydroxy group.

Said lactones are preferably selected from the group consisting of tetrahydro-3-hydroxy-5-oxo-3-furanacetic acid, tetrahydro-3-hydroxy-2-oxo-3-furanacetic acid, tetrahydro-5-oxo-3-furanacetic acid and tetrahydro-2-oxo-3-furanacetic acid.

A product as defined above is obtainable by the above-defined method, preferably by one of the above-defined preferred methods.

The present invention also relates to the compound tetrahydro-3-hydroxy-2-oxo-3-furanacetic acid 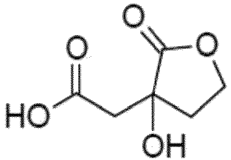 which has not been described before.

The present application also relates to the use of one or more compounds selected from the group consisting of aliphatic tricarboxylic acids, esters thereof, anhydrides thereof, and salts thereof, as starting compound(s) for making
- one or more product compounds selected from the group consisting of
   - lactones having one carboxyl group and one hydroxy group, and
   - lactones having one carboxyl group and no hydroxy group,
   or
- mixtures of such product compounds.

Regarding specific and preferred aliphatic tricarboxylic acids, esters thereof, anhydrides thereof, and salts thereof to be used as starting compounds for making the above-defined product compounds, the same applies as disclosed in the context of the above-defined method. Most preferred is the use of citric acid and aconitic acid as starting compounds for making the above-defined product.

Regarding specific and preferred product compounds, the same applies as disclosed in the context of the above-defined method. Lactones having one carboxyl group and one hydroxy group are preferably selected from the group consisting of tetrahydro-3-hydroxy-5-oxo-3-furanacetic acid and tetrahydro-3-hydroxy-2-oxo-3-furanacetic acid. Lactones having one carboxyl group and no hydroxy group are preferably selected from the group consisting of tetrahydro-5-oxo-3-furanacetic acid and tetrahydro-2-oxo-3-furanacetic acid.

Preferred is the use of one or more compounds selected from the group consisting of aliphatic tricarboxylic acids, esters thereof, anhydrides thereof, and salts thereof, as starting compound(s) for making
- product compounds selected from the group consisting of
   - lactones having one carboxyl group and one hydroxy group, and
   - lactones having one carboxyl group and no hydroxy group,
   or
- mixtures of such product compounds
in quantities of equal to or more than 100 kg, more preferably equal to or more than 500 kg and even more preferably equal to or more than 1000 kg per batch.

Most preferably the above-defined preferred compounds selected from the group consisting of aliphatic tricarboxylic acids, esters thereof, anhydrides thereof, and salts thereof are used as starting compound(s) in the method as defined above.

The present application also relates to the use of a heterogeneous hydrogenation catalyst comprising one or more metals selected from the group consisting of Co, Rh, Ir, Ni, Pd and Pt, in a total amount of 90 wt.-% or more, preferably 95 wt.-% or more, based on the total amount of the heterogeneous hydrogenation catalyst, wherein preferably the total amount of Pd and Rh is 90 wt.-% or more, preferably 95 wt.-% or more, wherein more preferably the amount of Pd or Rh is 90 wt.-% or more, preferably 95 wt.-% or more, in a method of preparing a product comprising one, two or more product compounds selected from the group consisting of lactones having at least one group selected from carboxyl, carboalkoxy, hydroxy and carboxylate, wherein the method comprises chemically converting one, two or more starting compounds selected from the group consisting of aliphatic tricarboxylic acids, esters thereof, anhydrides thereof, and salts thereof so that a product comprising one, two or more product compounds selected from the group consisting of lactones having at least one group selected from carboxyl, carboalkoxy, hydroxy and carboxylate results.

When the above-defined heterogeneous hydrogenation catalyst is used with an aliphatic tricarboxylic acid or a salt thereof or the anhydride thereof as a starting compound, said starting compound is preferably chemically converted in step (iii) so that predominantly the number of carbon atoms present in said starting compound is maintained so that the resulting lactone(s) has/have the same number of carbon atoms as said starting compound. Thus, predominantly no carbon atom of the starting compound is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When the above-defined heterogeneous hydrogenation catalyst is used with an aliphatic tricarboxylic acid having a number of carbon atoms of 6 or a salt thereof or the anhydride thereof as a starting compound, said starting compound is preferably chemically converted in step (iii) so that predominantly the number of carbon atoms present in said starting compound is maintained so that the resulting lactone(s) has/have 6 carbon atoms including a carboxyl group. Thus, predominantly no carbon atom of the starting compound is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having 6 carbon atoms, residual starting compounds, and product compounds formed by side reactions which are not lactones having 6 carbon atoms.

When the above-defined heterogeneous hydrogenation catalyst is used with an ester of an aliphatic tricarboxylic acid as a starting compound, said starting compound is preferably chemically converted in step (iii) so that predominantly the number of carbon atoms present in the aliphatic tricarboxylic acid corresponding to said ester is maintained so that the resulting lactone(s) either has/have the a number of carbon atoms which is the sum of the number of carbon atoms in the aliphatic tricarboxylic acid corresponding to the ester used as the starting compound and the number of carbon atoms in the remaining carboalkoxy group (which is not involved in formation of the lactone group) of the starting compound, or in case the carboalkoxy group of the starting compound which is not involved in formation of the lactone group is reduced to a hydroxy group under the above-defined hydrogenation conditions, the resulting lactone(s) has/have the same number of carbon atoms as said aliphatic tricarboxylic acid corresponding to the ester used as the starting compound. Thus, predominantly no carbon atom of the aliphatic tricarboxylic acid corresponding to said ester used as the starting compound is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having at least one group selected from carboxyl, carboalkoxy, hydroxy and carboxylate, residual starting compounds, and product compounds formed by side reactions which are not lactones having at least one group selected from carboxyl, carboalkoxy, hydroxy and carboxylate.

When the above-defined heterogeneous hydrogenation catalyst is used with an ester of an aliphatic tricarboxylic acid having a number of carbon atoms of 6 as a starting compound, said starting compound is preferably chemically converted in step (iii) so that predominantly the number of carbon atoms present in the aliphatic tricarboxylic acid corresponding to said ester is maintained so that the resulting lactone(s) either has/have a number of carbon atoms which is the sum of 6 and the number of carbon atoms in the remaining carboalkoxy group (which is not involved in formation of the lactone group) of the starting compound, or in case the carboalkoxy group not involved in formation of the lactone group is reduced to a hydroxy group under the above-defined hydrogenation conditions, the resulting lactone(s) has/have 6 carbon atoms. Thus, predominantly no carbon atom of the aliphatic tricarboxylic acid corresponding to the ester used as the starting compound is lost by decarboxylation. Herein, predominantly means that more than 50% by weight of the above-defined product (a) fulfills the above-defined condition. Preferably, more than 60%, more preferably more than 70% by weight of the product (a) fulfills the above-defined condition. Herein, the total weight of the product (a) is the sum of the weights of the lactones having 6 carbon atoms including a carboxyl group, residual starting com-pounds, and product compounds formed by side reactions which are not lactones having at least one group selected from carboxyl, carboalkoxy, hydroxy and carboxylate.

Regarding specific and preferred heterogeneous hydrogenation catalysts, the same applies as disclosed in the context of the above-defined method. Regarding specific and preferred aliphatic tricarboxylic acids, esters thereof, anhydrides thereof, and salts thereof to be converted in the method using the above-defined catalyst, the same applies as disclosed in the context of the above-defined method. Most preferred is the use of citric acid and aconitic acid for making the above-defined product.

Regarding specific and preferred product compounds, the same applies as disclosed in the context of the above-defined method. Lactones having one carboxyl group and one hydroxy group are preferably selected from the group consisting of tetrahydro-3-hydroxy-5-oxo-3-furanacetic acid and tetrahydro-3-hydroxy-2-oxo-3-furanacetic acid. Lactones having one carboxyl group and no hydroxy group are preferably selected from the group consisting of tetrahydro-5-oxo-3-furanacetic acid and tetrahydro-2-oxo-3-furanacetic acid.

Regarding specific and preferred reaction parameters (temperature, hydrogen partial pressure, duration of chemically conversion, solvent), the same applies as disclosed in the context of the above-defined method. Most preferably, the above-defined heterogeneous catalyst is used in the method as defined above.

### Examples:

The following examples according to the present invention are meant to further explain and illustrate the present invention without limiting its scope.

Fig. 1 shows formation of lactones having a carboxyl group from citric acid as well the undesired complete conversion of the carboxyl groups of citric acid to hydroxy groups.

Fig. 2 shows the formation of lactones having a carboxyl group and a hydroxy group from citric acid by reduction of a carboxyl group to a hydroxy group which then undergoes a ring-closing reaction (intramolecular esterification) with another carboxyl group of the same molecule so that a lactone having one a carboxyl group is obtained, and subsequent dehydration and hydrogenation to lactones having a carboxyl group and no hydroxy group.

Preparation of lactones having a carboxyl group from citric acid dissolved in water was carried out as follows:
The heterogeneous hydrogenation catalyst (type of catalyst, type of support material and amount given in table 1) was added to a solution of citric acid monohydrate in water as the solvent (citric acid concentration and amount of solution given in table 1) in an autoclave. The reaction vessel was closed and then rinsed twice with nitrogen gas (0.5 MPa). Then stirring (700 U/min) and initial hydrogen pressure (5 MPa) were applied. The reaction mixture was heated up to the temperature given in table 1, and the hydrogen pressure was increased up to the value given in table 1. The reaction mixture was stirred under these conditions for the time period given in table 1, then cooled to room temperature and rinsed twice with nitrogen gas (0.5 MPa). Afterwards, the catalyst was filtered off and the solvent was removed by evaporation. The resulting oil was analyzed by gas chromatography and HPLC. The experimental parameters and results of the examples with citric acid are compiled in table 1.

Preparation of lactones having a carboxyl group from aconitic acid dissolved in water was carried out as follows:
The heterogeneous hydrogenation catalyst (type of catalyst, type of support material and amount given in table 2) was added to a solution of aconitic acid in water as the solvent (aconitic acid concentration and amount of solution given in table 2) in an autoclave. The reaction vessel was closed and then rinsed twice with nitrogen gas (0.5 MPa). Then stirring (UPM given in table 2) and initial hydrogen pressure (5 MPa) were applied. The reaction mixture was heated up to the temperature given in table 2, and the hydrogen pressure was increased up to the value given in table 2. The reaction mixture was stirred under these conditions for the time period given in table 2, then cooled to room temperature and rinsed twice with nitrogen gas (0.5 MPa). Afterwards, the catalyst was filtered off and the solvent was removed by evaporation. The resulting oil was analyzed by gas chromatography and HPLC. The experimental parameters and results of the examples with aconitic acid are compiled in table 2.

Preparation of lactones having a carboxyl group from tricarballylic acid dissolved in water was carried out as follows:
The heterogeneous hydrogenation catalyst (type of catalyst, type of support material and amount given in table 3) was added to a solution of tricarballylic acid in water as the solvent (tricarballylic acid concentration and amount of solution given in table 3) in an autoclave. The reaction vessel was closed and then rinsed twice with nitrogen gas (0.5 MPa). Then stirring (UPM given in table 3) and initial hydrogen pressure (5 MPa) were applied. The reaction mixture was heated up to the temperature given in table 3, and the hydrogen pressure was increased up to the value given in table 3. The reaction mixture was stirred under these conditions for the time period given in table 3, then cooled to room temperature and rinsed twice with nitrogen gas (0.5 MPa). Afterwards, the catalyst was filtered off and the solvent was removed by evaporation. The resulting oil was analyzed by gas chromatography and HPLC. The experimental parameters and results of the examples with tricarballylic acid are compiled in table 3.

In the tables,
- lactones include tetrahydro-3-hydroxy-5-oxo-3-furanacetic acid, tetrahydro-3-hydroxy-2-oxo-3-furanacetic acid, tetrahydro-5-oxo-3-furanacetic acid, and tetrahydro-2-oxo-3-furanacetic acid,
- C₆-polyolols include 3-(hydroxymethyl)pentane-1,3,5-triol, propane-1,2,3-trimethanol and 3-(hydroxymethyl)-2-pentene-1,5-diol.
- C₅-triol is 1,3,5-pentanetriol.

In each experiment, a significant fraction of lactones was obtained.

**Table 1**

| Parameters and Results of Examples with Citric Acid | | | | | | | | | | | |
|---|---|---|---|---|---|---|---|---|---|---|---|
| No. | weight of catalyst relative to the weight of support material + catalyst | conc. of citric acid dissolved in H₂O [wt%] | amount of aq. citric acid solution [g] | amount of catalyst + support material [g] | time [h] | p (H₂) [MPa] | T [°C] | Composition of resulting oil | | | |
| | | | | | | | | Citric Acid [wt%] | C₆- and C₅-polyols [wt%] | C₆-lactones [wt%] | Others [wt%] |
| 1 | 10% Pd/C | 25 | 100 | 10 | 20 | 25 | 100 | 23 | - | 71 | 6 |
| 2 | 10% Pd/C | 54 | 100 | 10 | 20 | 25 | 120 | 23 | 3 | 71 | 3 |
| 3 | 10% Pd/C | 54 | 100 | 10 | 48 | 18 | 100 | 31 | 1 | 65 | 3 |
| 4 | 10% Pd/C | 54 | 100 | 10 | 72 | 18 | 100 | 15 | 3 | 77 | 1 |
| 5 | 10% Pd/C | 54 | 120 | 12 | 72 | 18 | 100 | 10 | 1 | 82 | 7 |
| 6 | 5% Rh/Al2O3 | 54 | 100 | 2.5 | 120 | 27.5 | 110 | 23 | - | 74 | 3 |
| 7 | 10% Pd/C | 54 | 100 | 10 | 48 | 18 | 120 | 2 | 1 | 91 | 6 |

**Table 2**

| Parameters and Results of Examples with Aconitic Acid | | | | | | | | | | | | |
|---|---|---|---|---|---|---|---|---|---|---|---|---|
| No. | weight of catalyst relative to the weight of support material + catalyst | conc. of aconitic acid dissolved in H₂O [wt%] | amount of aq. aconitic acid solution [g] | amount of catalyst + support material [g] | UPM | time [h] | p (H₂) [MPa] | T [°C] | Composition of resulting oil | | | |
| | | | | | | | | | Aconitic Acid [wt%] | C₅- and C₆-polyols | C₆-lactones [wt%] | Others [wt%] |
| 1 | 10 wt% Pd/C | 25 | 100 | 5 | 700 | 60 | 25 | 100 | 7 | - | 85 | 8 |
| 2 | 10 wt% Pd/C | 25 | 100 | 10 | 700 | 60 | 25 | 100 | 6 | - | 84 | 10 |
| 3 | 10 wt% Pd/C | 25 | 500 | 50 | 1000 | 72 | 25 | 100 | 1 | - | 84 | 15 |

**Table 3**

| Parameters and Results of Examples with Tricarballylic Acid | | | | | | | | | | | |
|---|---|---|---|---|---|---|---|---|---|---|---|
| No. | weight of catalyst relative to the weight of support material + catalyst | conc. of tricarballylic acid dissolved in H₂O [wt%] | amount of aq. tricarballylic acid solution [g] | amount of catalyst + support material [g] | time [h] | p (H₂) [MPa] | T [°C] | Composition of resulting oil | | | |
| | | | | | | | | Tricarballylic Acid [wt%] | C₅- and C₆-Polyols | C₆-lactones [wt%] | Others [wt%] |
| 1 | 10 wt% Pd/C | 25 | 100 | 10 | 60 | 25 | 100 | 4 | - | 83 | 13 |

## Claims

1. Method of preparing a product comprising
(a) one, two or more product compounds selected from the group consisting of lactones having at least one group selected from carboxyl, carboalkoxy, hydroxy and carboxylate
or
(b) one or more reaction products of said one, two or more compounds selected from the group consisting of lactones having at least one group selected from carboxyl, carboalkoxy, hydroxy and carboxylate
comprising at least the following steps:
(i) providing or preparing a starting material comprising one, two or more starting compounds selected from the group consisting of aliphatic tricarboxylic acids having a total number of carbon atoms of 6, esters thereof, anhydrides thereof, and salts thereof,
(ii) providing or preparing a solvent having a dielectric constant above the dielectric constant of n-butanol,
and
(iii) chemically converting said one, two or more starting compounds provided or prepared in step (i)
- at a temperature in the range of from 80 °C to 140 °C,
- at a partial pressure of hydrogen in the range of from 15 MPa to 30 MPa,
- in said solvent provided or prepared in step (ii), and
- in the presence of a heterogeneous hydrogenation catalyst comprising one or more metals selected from the group consisting of Co, Rh, Ir, Ni, Pd and Pt,
so that two carboxyl groups, two carboxylate groups, two carboalkoxy groups, or the anhydride group as present in said one, two or more starting compounds under hydrogenation conditions form a lactone group so that a product comprising one, two or more product compounds selected from the group consisting of lactones having at least one group selected from carboxyl, carboalkoxy, hydroxy and carboxylate results
so that
- when the starting compound is an aliphatic tricarboxylic acid, under hydrogenation conditions as defined above two of its carboxyl groups selectively form a lactone group, and one carboxyl group remains,
- when the starting compound is a salt of an aliphatic tricarboxylic acid, under hydrogenation conditions as defined above two of its carboxylate groups selectively form a lactone group, and one carboxylate group remains,
- when the starting compound is an ester of an aliphatic tricarboxylic acid, under hydrogenation conditions as defined above two of its carboalkoxy groups selectively form a lactone group, and one carboalkoxy group remains or is reduced to a hydroxy group under the above-defined hydrogenation conditions,
- when the starting compound is an anhydride of an aliphatic tricarboxylic acid, under hydrogenation conditions as defined above from the anhydride group a lactone is formed selectively, and one carboxyl group remains.

2. Method according to claim 1, wherein
- the heterogeneous hydrogenation catalyst comprises one or more metals selected from the group consisting of Rh and Pd
and/or
- when the starting compound is an aliphatic tricarboxylic acid having a number of carbon atoms of 6 or a salt thereof or the anhydride thereof, the resulting lactone(s) has/have 6 carbon atoms including a carboxyl group,
and when an ester of an aliphatic tricarboxylic acid having a number of carbon atoms of 6 is used as a starting compound, the resulting lactone(s) either has/ have a number of carbon atoms which is the sum of 6 and the number of carbon atoms in the remaining carboalkoxy group which is not involved in formation of the lactone group, or in case the carboalkoxy group not involved in formation of the lactone group is reduced to a hydroxy group, the resulting lactone(s) has/have 6 carbon atoms.

3. Method according to any preceding claim, wherein the one starting compound or at least one of the two or more starting compounds, respectively,
- is selected from the group consisting of alpha-functionalized aliphatic tricarboxylic acids, esters thereof, anhydrides thereof, and salts thereof, preferably from the group consisting of alpha-hydroxy aliphatic tricarboxylic acids, esters thereof, anhydrides thereof, and salts thereof, more preferably from the group consisting of citric acid, isocitric acid, esters thereof, anhydrides thereof, and salts thereof, most preferably from the group consisting of citric acid, triethyl citrate, and isocitric acid,
or
- is selected from the group consisting of alpha-beta-unsaturated aliphatic tricarboxylic acids, esters thereof, anhydrides thereof, and salts thereof, preferably from the group consisting of aconitic acid, esters thereof, anhydrides thereof, and salts thereof,
or
- is selected from the group consisting of tricarballylic acid, esters thereof, anhydrides thereof, and salts thereof.

4. Method according to any preceding claim, wherein the product resulting in step (iii) comprises one, two or more product compounds selected from the group consisting of
- lactones having one carboxyl group and one hydroxy group, preferably selected from the group consisting of tetrahydro-3-hydroxy-5-oxo-3-furanacetic acid and tetrahydro-3-hydroxy-2-oxo-3-furanacetic acid
and
- lactones having one carboxyl group and no hydroxy group, preferably selected from the group consisting of tetrahydro-5-oxo-3-furanacetic acid and tetrahy-dro-2-oxo-3-furanacetic acid.

5. Method according to any preceding claim, wherein the solvent having a dielectric constant above the dielectric constant of n-butanol provided or prepared in step (ii) comprises one or more constituents selected from the group consisting of water, methanol, ethanol, n-propanol, iso-propanol, ethylene glycols, propylene glycols and cyclic ethers,
wherein the solvent is preferably selected from the group consisting of
- water,
and
- aqueous mixtures comprising water in an amount of more than 50 wt.-%, preferably more than 70 wt.-%, more preferably more than 90 wt.-%, based on the total amount of the solvent.

6. Method according to any preceding claim, wherein the temperature in step (iii) is
- in the range of from 80 °C to 125 °C, preferably in the range of from 95 °C to 120 °C,
and/or
- chosen so as to selectively prepare said lactones having at least one group selected from carboxyl, carboalkoxy, hydroxy and carboxylate, instead of aliphatic polyols.

7. Method according to any preceding claim, wherein the heterogeneous hydrogenation catalyst comprises one or more metals selected from the group consisting of Co, Rh, Ir, Ni, Pd and Pt in a total amount of 90 wt.-% or more, preferably 95 wt.-% or more, based on the total amount of the heterogeneous hydrogenation catalyst, wherein preferably the heterogeneous hydrogenation catalyst is supported by a support material, wherein preferably the support material is selected from the group consisting of metal oxides, zeolites and carbon-based materials, preferably selected from the group consisting of Al₂O₃, ZrO₂, TiO₂, SiC, carbon black and PTFE.

8. Method according to any preceding claim, wherein one, two or all of the following additional steps are carried out after step (iii):
- removing solvent used in step (iii) by evaporation,
- chemically converting one, two or more of said product compounds selected from the group consisting of lactones having at least one group selected from carboxyl, carboalkoxy, hydroxy and carboxylate present in the product resulting from step (iii) to give a product (b) comprising one or more reaction products of said one, two or more compounds selected from the group consisting of lactones having at least one group selected from carboxyl, carboalkoxy, hydroxy and carboxylate,
and
- adding one or more additional chemical substances to one, two or more of said product compounds present in the product resulting from step (iii) selected from the group consisting of lactones having at least one group selected from carboxyl, carboalkoxy, hydroxy and carboxylate so that a reaction mixture results comprising said one, two or more compounds selected from the group consisting of lactones having at least one group selected from carboxyl, carboalkoxy, hydroxy and carboxylate.

9. Method according to any preceding claim, wherein the starting material comprising one, two or more starting compounds selected from the group consisting of aliphatic tricarboxylic acids, esters thereof, anhydrides thereof, and salts thereof, preferably selected from the group consisting of citric acid and aconitic acid, is prepared or isolated from plant material, preferably prepared by sugar fermentation.

10. Method according to any preceding claim, wherein the product comprises
(a) one, two or more product compounds selected from the group consisting of lactones having at least one group selected from carboxyl, carboalkoxy, hydroxy and carboxylate
wherein said product is selected from the group consisting of
- formulations comprising one or more materials selected from the group consisting of peptides, proteins, enzymes, microorganisms, DNA, RNA, and viruses
- reaction mixtures for preparing polymers, preferably for preparing polymers by ring-opening polymerizations or by alkoxylations with ethylene oxide and/or propylene oxide, wherein the reaction mixture is prepared after step (iii),
- mixtures comprising metal cations, preferably Fe, Mg, Ca, Sr, Cu, Ag and Au cations, complexed by said one, two or more product compounds selected from the group consisting of lactones having at least one group selected from carboxyl, carboalkoxy, hydroxy and carboxylate, wherein the mixture is prepared after step (iii), wherein the mixture is preferably selected from the group consisting of refinery products, mining products, and home care products,
- reaction mixtures for preparing amides or esters of said one, two or more product compounds selected from the group consisting of lactones having at least one group selected from carboxyl, carboalkoxy, hydroxy and carboxylate, wherein the reaction mixture is prepared after step (iii)
or
(b) one or more reaction products of said one, two or more product compounds selected from the group consisting of lactones having at least one group selected from carboxyl, carboalkoxy, hydroxy and carboxylate,
wherein said product is selected from the group consisting of
- products comprising one or more polymers, preferably polyalkoxylates or polyesters, wherein at least one of said polymers is prepared from said one, two or more product compounds selected from the group consisting of lactones having at least one group selected from carboxyl, carboalkoxy, hydroxy and carboxylate, in an additional step conducted after step (iii),
- solutions comprising as a solvent or solvent constituent one or more reaction products of said one, two or more product compounds selected from the group consisting of lactones having at least one group selected from carboxyl, carboalkoxy, hydroxy and carboxylate.

11. Tetrahydro-3-hydroxy-2-oxo-3-furanacetic acid.

12. Use of one, two or more compounds selected from the group consisting of aliphatic tricarboxylic acids, esters thereof, anhydrides thereof, and salts thereof, preferably use of citric acid, as starting compound(s) in a method according to any of claims 1 to 10 for making
- product compounds selected from the group consisting of
- lactones having one carboxyl group and one hydroxy group, preferably selected from the group consisting of tetrahydro-3-hydroxy-5-oxo-3-furanacetic acid and tetrahydro-3-hydroxy-2-oxo-3-furanacetic acid
and
- lactones having one carboxyl group and no hydroxy group, preferably selected from the group consisting of tetrahydro-5-oxo-3-furanacetic acid and tetrahydro-2-oxo-3-furanacetic acid,
or
- mixtures of such product compounds,
wherein preferably these product compound(s) or mixtures of such product compounds are made in quantities of equal to or more than 100 kg, more preferably equal to or more than 500 kg and even more preferably equal to or more than 1000 kg per batch.

13. Use of a heterogeneous hydrogenation catalyst comprising one or more metals selected from the group consisting of Co, Rh, Ir, Ni, Pd and Pt, in a total amount of 90 wt.-% or more, preferably 95 wt.-% or more, based on the total amount of the heterogeneous hydrogenation catalyst,
in a method according to any of claims 1 to 10 of preparing a product comprising one, two or more product compounds selected from the group consisting of lactones having at least one group selected from carboxyl, carboalkoxy, hydroxy and carboxylate,
preferably comprising one, two or more product compounds selected from the group consisting of
- lactones having one carboxyl group and one hydroxy group, preferably selected from the group consisting of tetrahydro-3-hydroxy-5-oxo-3-furanacetic acid and tetrahydro-3-hydroxy-2-oxo-3-furanacetic acid
and
- lactones having one carboxyl group and no hydroxy group, preferably selected from the group consisting of tetrahydro-5-oxo-3-furanacetic acid and tetrahydro-2-oxo-3-furanacetic acid.

14. Use according to claim 13, wherein the heterogeneous hydrogenation catalyst comprises one or more metals selected from the group consisting of Rh and Pd.

## Patentansprüche

1. Verfahren zum Herstellen eines Produkts, umfassend:
(a) eine, zwei oder mehr Produktverbindungen ausgewählt aus der Gruppe bestehend aus Lactonen mit mindestens einer Gruppe ausgewählt aus Carboxyl, Carboalkoxy, Hydroxy und Carboxylat
oder
(b) ein oder mehrere Reaktionsprodukte der einen, zwei oder mehr Verbindungen ausgewählt aus der Gruppe bestehend aus Lactonen mit mindestens einer Gruppe ausgewählt aus Carboxyl, Carboalkoxy, Hydroxy und Carboxylat,
umfassend mindestens die folgenden Schritte:
(i) Bereitstellen oder Herstellen eines Ausgangsmaterials umfassend eine, zwei oder mehr Ausgangsverbindungen ausgewählt aus der Gruppe bestehend aus aliphatischen Tricarbonsäuren mit einer Gesamtzahl von Kohlenstoffatomen von 6, Estern davon, Anhydriden davon und Salzen davon,
(ii) Bereitstellen oder Herstellen eines Lösungsmittels mit einer Dielektrizitätskonstante über der Dielektrizitätskonstante von n-Butanol
und
(iii) chemisches Umwandeln der einen, zwei oder mehr Ausgangsverbindungen, die in Schritt (i) bereitgestellt oder hergestellt wurden
- bei einer Temperatur im Bereich von 80 °C bis 140 °C,
- bei einem Wasserstoff-Partialdruck im Bereich von 15 MPa bis 30 MPa,
- in dem Lösungsmittel, das in Schritt (ii) bereitgestellt oder hergestellt wurde, und
- in Gegenwart eines heterogenen Hydrierkatalysators umfassend ein oder mehrere Metalle ausgewählt aus der Gruppe bestehend aus Co, Rh, Ir, Ni, Pd und Pt,
so dass zwei Carboxylgruppen, zwei Carboxylatgruppen, zwei Carboalkoxygruppen oder die Anhydridgruppe, wie in der einen, den zwei oder mehr Ausgangsverbindungen vorhanden, unter Hydrierungsbedingungen eine Lactongruppe bilden, so dass ein Produkt entsteht, das eine, zwei oder mehr Produktverbindungen ausgewählt aus der Gruppe bestehend aus Lactonen mit mindestens einer Gruppe ausgewählt aus Carboxyl, Carboalkoxy, Hydroxy und Carboxylat umfasst,
so dass
- wenn die Ausgangsverbindung eine aliphatische Tricarbonsäure ist, unter wie oben definierten Hydrierungsbedingungen zwei ihrer Carboxylgruppen selektiv eine Lactongruppe bilden und eine Carboxylgruppe verbleibt,
- wenn die Ausgangsverbindung ein Salz einer aliphatischen Tricarbonsäure ist, unter wie oben definierten Hydrierungsbedingungen zwei ihrer Carboxylatgruppen selektiv eine Lactongruppe bilden und eine Carboxylatgruppe verbleibt,
- wenn die Ausgangsverbindung ein Ester einer aliphatischen Tricarbonsäure ist, unter wie oben definierten Hydrierungsbedingungen zwei ihrer Carboalkoxygruppen selektiv eine Lactongruppe bilden und eine Carboalkoxygruppe unter den oben definierten Hydrierungsbedingungen verbleibt oder zu einer Hydroxygruppe reduziert wird,
- wenn die Ausgangsverbindung ein Anhydrid einer aliphatischen Tricarbonsäure ist, unter wie oben definierten Hydrierungsbedingungen aus der Anhydridgruppe selektiv ein Lacton gebildet wird und eine Carboxylgruppe verbleibt.

2. Verfahren nach Anspruch 1, wobei
- der heterogene Hydrierkatalysator ein oder mehrere Metalle ausgewählt aus der Gruppe bestehend aus Rh und Pd umfasst
und/oder,
- wenn die Ausgangsverbindung eine aliphatische Tricarbonsäure mit einer Anzahl von Kohlenstoffatomen von 6 oder eine Salz davon oder das Anhydrid davon ist, das(die) erhaltene(n) Lacton(e) 6 Kohlenstoffatome einschließlich einer Carboxylgruppe aufweist/aufweisen, und wenn ein Ester einer aliphatischen Tricarbonsäure mit einer Anzahl von Kohlenstoffatomen von 6 als eine Ausgangsverbindung verwendet wird, das(die) erhaltene(n) Lacton(e) entweder eine Anzahl von Kohlenstoffatomen aufweist (aufweisen), welche die Summe von 6 ist, und die Anzahl von Kohlenstoffatomen in der verbleibenden Carboalkoxygruppe nicht an der Bildung der Lactongruppe beteiligt ist, oder falls die an der Bildung der Lactongruppe nicht beteiligte Carboalkoxygruppe zu einer Hydroxygruppe reduziert wird, das(die) erhaltene(n) Lacton(e) 6 Kohlenstoffatome aufweist(aufweisen).

3. Verfahren nach einem vorhergehenden Anspruch, wobei die eine Ausgangsverbindung bzw. mindestens eine der zwei oder mehr Ausgangsverbindungen,
- ausgewählt ist aus der Gruppe bestehend aus alphafunktionalisierten aliphatischen Tricarbonsäuren, Estern davon, Anhydriden davon und Salzen davon, vorzugsweise aus der Gruppe bestehend aus alpha-hydroxyaliphatischen Tricarbonsäuren, Estern davon, Anhydriden davon und Salzen davon, besonders bevorzugt aus der Gruppe bestehend aus Zitronensäure, Isozitronensäure, Estern davon, Anhydriden davon und Salzen davon, ganz besonders bevorzugt aus der Gruppe bestehend aus Zitronensäure, Triethylzitrat und Isozitronensäure,
oder
- ausgewählt ist aus der Gruppe bestehend aus alpha-beta-ungesättigten aliphatischen Tricarbonsäuren, Estern davon, Anhydriden davon und Salzen davon, vorzugsweise aus der Gruppe bestehend aus Aconitsäure, Estern davon, Anhydriden davon und Salzen davon,
oder
- ausgewählt ist aus der Gruppe bestehend aus Tricarballylsäure, Estern davon, Anhydriden davon und Salzen davon.

4. Verfahren nach einem vorhergehenden Anspruch, wobei das Produkt, das in Schritt (iii) entsteht, eine, zwei oder mehr Produktverbindungen umfasst, die ausgewählt sind aus der Gruppe bestehend aus
- Lactonen mit einer Carboxylgruppe und einer Hydroxygruppe, vorzugsweise ausgewählt aus der Gruppe bestehend aus Tetrahydro-3-hydroxy-5-oxo-3-furanessigsäure und Tetrahydro-3-hydroxy-2-oxo-3-furanessigsäure
und
- Lactonen mit einer Carboxylgruppe und keiner Hydroxygruppe, vorzugsweise ausgewählt aus der Gruppe bestehend aus Tetrahydro-5-oxo-3-furanessigsäure und Tetrahydro-2-oxo-3-furanessigsäure.

5. Verfahren nach einem vorhergehenden Anspruch, wobei das Lösungsmittel mit einer Dielektrizitätskonstante über der Dielektrizitätskonstante von n-Butanol, das in Schritt (ii) bereitgestellt oder hergestellt wird, einen oder mehrere Bestandteile ausgewählt aus der Gruppe bestehend aus Wasser, Methanol, Ethanol, n-Propanol, Isopropanol, Ethylenglykolen, Propylenglykolen und cyclischen Ethern umfasst,
wobei das Lösungsmittel vorzugsweise ausgewählt ist aus der Gruppe bestehend aus
- Wasser
und
- wässrigen Mischungen, die Wasser in einer Menge von mehr als 50 Masse-%, vorzugsweise mehr als 70 Masse-%, besonders bevorzugt mehr als 90 Masse-%, bezogen auf die Gesamtmenge des Lösungsmittels, umfassen.

6. Verfahren nach einem vorhergehenden Anspruch, wobei die Temperatur in Schritt (iii)
- im Bereich von 80 °C bis 125 °C, vorzugsweise im Bereich von 95 °C bis 120 °C, liegt
und/oder,
- ausgewählt ist, um selektiv Lactone herzustellen, die mindestens eine Gruppe ausgewählt aus Carboxyl, Carboalkoxy, Hydroxy und Carboxylat aufweisen, anstelle von aliphatischen Polyolen.

7. Verfahren nach einem vorhergehenden Anspruch, wobei der heterogene Hydrierkatalysator ein oder mehrere Metalle ausgewählt aus der Gruppe bestehend aus Co, Rh, Ir, Ni, Pd und Pt in einer Gesamtmenge von 90 Masse-% oder mehr, vorzugsweise 95 Masse-% oder mehr, bezogen auf die Gesamtmenge des heterogenen Hydrierkatalysators, umfasst,
wobei vorzugsweise der heterogene Hydrierkatalysator von einem Trägermaterial getragen wird, wobei vorzugsweise das Trägermaterial ausgewählt ist aus der Gruppe bestehend aus Metalloxiden, Zeolithen und Materialien auf Kohlenstoffbasis, vorzugsweise ausgewählt ist aus der Gruppe bestehend aus Al₂O₃, ZrO₂, TiO₂, SiC, Ruß und PTFE.

8. Verfahren nach einem vorhergehenden Anspruch, wobei nach Schritt (iii) ein, zwei oder alle der folgenden zusätzlichen Schritte ausgeführt werden:
- Entfernen von in Schritt (iii) verwendetem Lösungsmittel durch Verdampfen,
- chemisches Umwandeln von einer, zwei oder mehr der Produktverbindungen ausgewählt aus der Gruppe bestehend aus Lactonen mit mindestens einer Gruppe ausgewählt aus Carboxyl, Carboalkoxy, Hydroxy und Carboxylat, die in dem Produkt, das in Schritt (iii) entstanden ist, vorhanden sind, zu einem Produkt (b), das ein oder mehrere Reaktionsprodukte der einen, zwei oder mehr Verbindungen ausgewählt aus der Gruppe bestehend aus Lactonen mit mindestens einer Gruppe ausgewählt aus Carboxyl, Carboalkoxy, Hydroxy und Carboxylat umfasst,
und
- Hinzufügen einer oder mehrerer zusätzlicher chemischer Substanzen zu einer, zwei oder mehr der Produktverbindungen, die in dem Produkt vorhanden sind, das bei Schritt (iii) entstanden ist, ausgewählt aus der Gruppe bestehend aus Lactonen mit mindestens einer Gruppe ausgewählt aus Carboxyl, Carboalkoxy, Hydroxy und Carboxylat, so dass eine Reaktionsmischung entsteht, welche die eine, zwei oder mehr Verbindungen ausgewählt aus der Gruppe bestehend aus Lactonen mit mindestens einer Gruppe ausgewählt aus Carboxyl, Carboalkoxy, Hydroxy und Carboxylat umfasst.

9. Verfahren nach einem vorhergehenden Anspruch, wobei das Ausgangsmaterial, das eine, zwei oder mehr Ausgangsverbindungen ausgewählt aus der Gruppe bestehend aus aliphatischen Tricarbonsäuren, Estern davon, Anhydriden davon und Salzen davon umfasst, bevorzugt ausgewählt aus der Gruppe bestehend aus Zitronensäure und Aconitsäure, aus Pflanzenmaterial hergestellt oder isoliert wird, bevorzugt durch Zuckerfermentation hergestellt wird.

10. Verfahren nach einem vorhergehenden Anspruch, wobei das Produkt umfasst
(a) eine, zwei oder mehr Produktverbindungen ausgewählt aus der Gruppe bestehend aus Lactonen mit mindestens einer Gruppe ausgewählt aus Carboxyl, Carboalkoxy, Hydroxy und Carboxylat
wobei das Produkt ausgewählt ist aus der Gruppe bestehend aus
- Formulierungen, die ein oder mehrere Materialien ausgewählt aus der Gruppe bestehend aus Peptiden, Proteinen, Enzymen, Mikroorganismen, DNS, RNS und Viren umfassen,
- Reaktionsmischungen zum Herstellen von Polymeren, vorzugsweise zum Herstellen von Polymeren durch ringöffnende Polymerisationen oder durch Alkoxylierungen mit Ethylenoxid und/oder Propylenoxid, wobei die Reaktionsmischung nach Schritt (iii) hergestellt wird,
- Mischungen umfassend Metallkationen, vorzugsweise Fe-, Mg-, Ca-, Sr-, Cu-, Ag- und Au-Kationen, die durch die eine, zwei oder mehr Produktverbindungen ausgewählt aus der Gruppe bestehend aus Lactonen mit mindestens einer Gruppe ausgewählt aus Carboxyl, Carboalkoxy, Hydroxy und Carboxylat komplexiert sind, wobei die Mischung nach Schritt (iii) hergestellt wird, wobei die Mischung vorzugsweise ausgewählt ist aus der Gruppe bestehend aus Raffinerieprodukten, Bergbauprodukten und Haushaltspflegeprodukten,
- Reaktionsmischungen zur Herstellung von Amiden oder Estern der einen, zwei oder mehr Produktverbindungen ausgewählt aus der Gruppe bestehend aus Lactonen mit mindestens einer Gruppe ausgewählt aus Carboxyl, Carboalkoxy, Hydroxy und Carboxylat, wobei die Reaktionsmischung nach Schritt (iii) hergestellt wird,
oder
(b) ein oder mehrere Reaktionsprodukte der einen, zwei oder mehr Produktverbindungen ausgewählt aus der Gruppe bestehend aus Lactonen mit mindestens einer Gruppe ausgewählt aus Carboxyl, Carboalkoxy, Hydroxy und Carboxylat,
wobei das Produkt ausgewählt ist aus der Gruppe bestehend aus
- Produkten, die ein oder mehrere Polymere, vorzugsweise Polyalkoxylate oder Polyester, umfassen, wobei mindestens eines der Polymere aus der einen, den zwei oder mehr Produktverbindungen ausgewählt aus der Gruppe bestehend aus Lactonen mit mindestens einer Gruppe ausgewählt aus Carboxyl, Carboalkoxy, Hydroxy und Carboxylat in einem zusätzlichen Schritt hergestellt wird, der nach Schritt (iii) ausgeführt wird,
- Lösungen, die als ein Lösungsmittel oder Lösungsmittelbestandteil ein oder mehrere Reaktionsprodukte der einen, der zwei oder mehr Produktverbindungen ausgewählt aus der Gruppe bestehend aus Lactonen mit mindestens einer Gruppeausgewählt aus Carboxyl, Carboalkoxy, Hydroxy und Carboxylat umfassen.

11. Tetrahydro-3-hydroxy-2-oxo-3-furanessigsäure.

12. Verwendung von einer, zwei oder mehr Verbindungen ausgewählt aus der Gruppe bestehend aus aliphatischen Tricarbonsäuren, Estern davon, Anhydriden davon und Salzen davon, vorzugsweise Verwendung von Zitronensäure, als Ausgangsverbindung(en) bei einem Verfahren nach einem der Ansprüche 1 bis 10 zum Herstellen von
- Produktverbindungen ausgewählt aus der Gruppe bestehend aus
- Lactonen mit einer Carboxylgruppe und einer Hydroxygruppe, vorzugsweise ausgewählt aus der Gruppe bestehend aus Tetrahydro-3-hydroxy-5-oxo-3-furanessigsäure und Tetrahydro-3-hydroxy-2-oxo-3-furanessigsäure
und
- Lactonen mit einer Carboxylgruppe und keiner Hydroxygruppe, vorzugsweise ausgewählt aus der Gruppe bestehend aus Tetrahydro-5-oxo-3-furanessigsäure und Tetrahydro-2-oxo-3-furanessigsäure,
oder
- Mischungen solcher Produktverbindungen,
wobei diese Produktverbindung(en) oder Mischungen solcher Produktverbindungen vorzugsweise in Mengen von gleich oder mehr als 100 kg, besonders bevorzugt gleich oder mehr als 500 kg und ganz besonders bevorzugt gleich oder mehr als 1000 kg pro Charge hergestellt werden.

13. Verwendung eines heterogenen Hydrierkatalysators, umfassend ein oder mehrere Metalle ausgewählt aus der Gruppe bestehend aus Co, Rh, Ir, Ni, Pd und Pt in einer Gesamtmenge von 90 Masse-% oder mehr, vorzugsweise 95 Masse-% oder mehr, bezogen auf die Gesamtmenge des heterogenen Hydrierkatalysators,
bei einem Verfahren nach einem der Ansprüche 1 bis 10 zum Herstellen eines Produkts umfassend eine, zwei oder mehr Produktverbindungen ausgewählt aus der Gruppe bestehend aus Lactonen mit mindestens einer Gruppe ausgewählt aus Carboxyl, Carboalkoxy, Hydroxy und Carboxylat,
vorzugsweise umfassend eine, zwei oder mehr Produktverbindungen ausgewählt aus der Gruppe bestehend aus
- Lactonen mit einer Carboxylgruppe und einer Hydroxygruppe, vorzugsweise ausgewählt aus der Gruppe bestehend aus Tetrahydro-3-hydroxy-5-oxo-3-furanessigsäure und Tetrahydro-3-hydroxy-2-oxo-3-furanessigsäure
und
- Lactonen mit einer Carboxylgruppe und keiner Hydroxygruppe, vorzugsweise ausgewählt aus der Gruppe bestehend aus Tetrahydro-5-oxo-3-furanessigsäure und Tetrahydro-2-oxo-3-furanessigsäure.

14. Verwendung nach Anspruch 13, wobei der heterogene Hydrierkatalysator ein oder mehrere Metalle ausgewählt aus der Gruppe bestehend aus Rh und Pd umfasst.

## Revendications

1. Procédé de préparation d'un produit comprenant
(a) un, deux ou plusieurs composés choisis dans le groupe constitué par les lactones comportant au moins un groupe choisi parmi carboxyle, carboalcoxy, hydroxy et carboxylate
ou
(b) un ou plusieurs produits de réaction desdits un, deux ou plusieurs composés choisis dans le groupe constitué par les lactones comportant au moins un groupe choisi parmi carboxyle, carboalcoxy, hydroxy et carboxylate
comprenant au moins les étapes suivantes :
(i) la fourniture ou la préparation d'une matière de départ comprenant un, deux ou plusieurs composés de départ choisis dans le groupe constitué par les acides tricarboxyliques aliphatiques comportant au total 6 atomes de carbone, des esters de ceux-ci, des anhydrides de ceux-ci, et des sels de ceux-ci,
(ii) la fourniture ou la préparation d'un solvant ayant une constante diélectrique supérieure à la constante diélectrique du n-butanol,
et
(iii) la conversion chimique desdits un, deux ou plusieurs composés de départ fournis ou préparés à l'étape (i)
- à une température comprise entre 80 °C et 140 °C,
- à une pression partielle d'hydrogène comprise entre 15 MPa et 30 MPa,
- dans ledit solvant fourni ou préparé à l'étape (ii),
et
- en présence d'un catalyseur d'hydrogénation hétérogène comprenant un ou plusieurs métaux choisis dans le groupe constitué par Co, Rh, Ir, Ni, Pd et Pt,
de sorte que deux groupes carboxyle, deux groupes carboxylate, deux groupes carboalcoxy ou le groupe anhydride présents dans lesdits un, deux ou plusieurs composés de départ, dans des conditions d'hydrogénation, forment un groupe lactone de sorte qu'un produit comprenant un, deux ou plusieurs composés choisis dans le groupe constitué par les lactones comportant au moins un groupe choisi parmi carboxyle, carboalcoxy, hydroxy et carboxylate soit obtenu
de sorte que
- lorsque le composé de départ est un acide tricarboxylique aliphatique, dans des conditions d'hydrogénation telles que définies ci-dessus, deux de ses groupes carboxyles forment sélectivement un groupe lactone, et il reste un groupe carboxyle,
- lorsque le composé de départ est un sel d'un acide tricarboxylique aliphatique, dans des conditions d'hydrogénation telles que définies ci-dessus, deux de ses groupes carboxylate forment sélectivement un groupe lactone, et il reste un groupe carboxylate.
- lorsque le composé de départ est un ester d'un acide tricarboxylique aliphatique, dans des conditions d'hydrogénation telles que définies ci-dessus, deux de ses groupes carboalcoxy forment sélectivement un groupe lactone, et il reste un groupe carboalcoxy ou celui-ci est réduit en groupe hydroxy dans les conditions d'hydrogénation définies ci-dessus,
- lorsque le composé de départ est un anhydride d'un acide tricarboxylique aliphatique, dans les conditions d'hydrogénation telles que définies ci-dessus, une lactone est formée sélectivement à partir du groupe anhydride, et il reste un groupe carboxyle.

2. Procédé selon la revendication 1, dans lequel
- le catalyseur d'hydrogénation hétérogène comprend un ou plusieurs métaux choisis dans le groupe constitué par Rh et Pd
et/ou
- lorsque le composé de départ est un acide tricarboxylique aliphatique ayant 6 atomes de carbone ou un sel de celui-ci ou l'anhydride de celui-ci, la ou les lactones obtenues ont 6 atomes de carbone incluant un groupe carboxyle,
et lorsqu'un ester d'un acide tricarboxylique aliphatique ayant 6 atomes de carbone est utilisé comme composé de départ, la ou les lactones obtenues ont soit un nombre d'atomes de carbone qui est la somme de 6 et du nombre d'atomes de carbone du groupe carboalcoxy restant qui n'intervient pas dans la formation du groupe lactone, soit, dans le cas où le groupe carboalcoxy non impliqué dans la formation du groupe lactone est réduit en groupe hydroxy, la ou les lactones obtenues ont 6 atomes de carbone.

3. Procédé selon l'une quelconque des revendications précédentes, dans lequel le composé de départ ou au moins un des deux ou plusieurs composés de départ, respectivement,
- est choisi dans le groupe constitué par les acides tricarboxyliques aliphatiques fonctionnalisés en alpha, les esters de ceux-ci, les anhydrides de ceux-ci, et les sels de ceux-ci, de préférence dans le groupe constitué par les acides tricarboxyliques alpha-hydroxyaliphatiques, les esters de ceux-ci, les anhydrides de ceux-ci, et les sels de ceux-ci, plus préférablement dans le groupe constitué par l'acide citrique, l'acide isocitrique, les esters de ceux-ci, les anhydrides de ceux-ci, et les sels de ceux-ci, le plus préférablement dans le groupe constitué par l'acide citrique, le citrate de triéthyle et l'acide isocitrique,
ou
- est choisi dans le groupe constitué par les acides tricarboxyliques aliphatiques alpha-bêta-insaturés, les esters de ceux-ci, les anhydrides de ceux-ci, et les sels de ceux-ci, de préférence dans le groupe constitué par l'acide aconitique, les esters de celui-ci, les anhydrides de celui-ci de ceux-ci, et les sels de celui-ci,
ou
- est choisi dans le groupe constitué par l'acide tricarballylique, les esters de celui-ci, les anhydrides de celui-ci et les sels de celui-ci.

4. Procédé selon l'une quelconque des revendications précédentes, dans lequel le produit résultant à l'étape (iii) comprend un, deux ou plusieurs composés choisis dans le groupe constitué par
- les lactones comportant un groupe carboxyle et un groupe hydroxy, de préférence choisies dans le groupe constitué par l'acide tétrahydro-3-hydroxy-5-oxo-3-furanacétique et l'acide tétrahydro-3-hydroxy-2-oxo-3-furanacétique
et
- les lactones comportant un groupe carboxyle et aucun groupe hydroxy, de préférence choisies dans le groupe constitué par l'acide tétrahydro-5-oxo-3-furanacétique et l'acide tétrahydro-2-oxo-3-furanacétique.

5. Procédé selon l'une quelconque des revendications précédentes, dans lequel le solvant ayant une constante diélectrique supérieure à la constante diélectrique du n-butanol fourni ou préparé à l'étape (ii) comprend un ou plusieurs constituants choisis dans le groupe constitué par l'eau, le méthanol, l'éthanol, le n-propanol, l'isopropanol, les éthylènes glycols, les propylènes glycols et les éthers cycliques,
dans lequel le solvant est de préférence choisi dans le groupe constitué par
- l'eau.
et
- des mélanges aqueux comprenant de l'eau en une quantité supérieure à 50 % en masse, de préférence supérieure à 70 % en masse, plus préférablement supérieure à 90 % en masse, par rapport à la quantité totale du solvant.

6. Procédé selon l'une quelconque des revendications précédentes, dans lequel la température à l'étape (iii) est
- comprise entre 80 °C et 125 °C, de préférence comprise entre 95 °C et 120 °C,
et/ou
- choisie de manière à préparer sélectivement lesdites lactones comportant au moins un groupe choisi parmi carboxyle, carboalcoxy, hydroxy et carboxylate, plutôt que les polyols aliphatiques.

7. Procédé selon l'une quelconque des revendications précédentes, dans lequel le catalyseur d'hydrogénation hétérogène comprend un ou plusieurs métaux choisis dans le groupe constitué par Co, Rh, Ir, Ni, Pd et Pt en une quantité totale supérieure ou égale à 90 % en masse, de préférence supérieure ou égale à 95 % en masse, par rapport à la quantité totale du catalyseur d'hydrogénation hétérogène,
dans lequel, de préférence, le catalyseur d'hydrogénation hétérogène est supporté par un matériau de support, de préférence, le matériau de support étant choisi dans le groupe constitué par les oxydes métalliques, les zéolites et les matériaux à base de carbone, de préférence choisi dans le groupe constitué par Al₂O₃, ZrO₂, TiO₂, SiC, le noir de carbone et le PTFE.

8. Procédé selon l'une quelconque des revendications précédentes, dans lequel une, deux ou la totalité des étapes supplémentaires suivantes sont réalisées après l'étape (iii) :
- l'élimination du solvant utilisé à l'étape (iii) par évaporation,
- la conversion chimique d'un, deux ou plusieurs desdits composés choisis dans le groupe constitué par les lactones comportant au moins un groupe choisi parmi carboxyle, carboalcoxy, hydroxy et carboxylate présent dans le produit issu de l'étape (iii) pour obtenir un produit (b) comprenant un ou plusieurs produits de réaction desdits un, deux ou plusieurs composés choisis dans le groupe constitué par les lactones comportant au moins un groupe choisi parmi carboxyle, carboalcoxy, hydroxy et carboxylate,
et
- l'ajout d'une ou plusieurs substances chimiques supplémentaires à un, deux ou plusieurs desdits composés présents dans le produit issu de l'étape (iii) choisis dans le groupe constitué par les lactones comportant au moins un groupe choisi parmi carboxyle, carboalcoxy, hydroxy et carboxylate de façon à obtenir un mélange réactionnel comprenant lesdits un, deux ou plusieurs composés choisis dans le groupe constitué par les lactones comportant au moins un groupe choisi parmi carboxyle, carboalcoxy, hydroxy et carboxylate.

9. Procédé selon l'une quelconque des revendications précédentes, dans lequel le matériau de départ comprenant un, deux ou plusieurs composés de départ choisis dans le groupe constitué par les acides tricarboxyliques aliphatiques, les esters de ceux-ci, les anhydrides de ceux-ci, et les sels de ceux-ci, de préférence choisis dans le groupe constitué par l'acide citrique et l'acide aconitique, est préparé ou isolé à partir de matériau végétal, de préférence préparé par fermentation de sucres.

10. Procédé selon l'une quelconque des revendications précédentes, dans lequel le produit comprend
(a) un, deux ou plusieurs composés choisis dans le groupe constitué par les lactones comportant au moins un groupe choisi parmi carboxyle, carboalcoxy, hydroxy et carboxylate
dans lequel ledit produit est choisi dans le groupe constitué par
- des formulations comprenant un ou plusieurs matériaux choisis dans le groupe constitué par les peptides, les protéines, les enzymes, les microorganismes, l'ADN, l'ARN et les virus,
- des mélanges réactionnels pour préparer des polymères, de préférence pour préparer des polymères par polymérisation par ouverture de cycle ou par alcoxylation avec de l'oxyde d'éthylène et/ou de l'oxyde de propylène, le mélange réactionnel étant préparé après l'étape (iii),
- des mélanges comprenant des cations métalliques, de préférence des cations Fe, Mg, Ca, Sr, Cu, Ag et Au, complexés par lesdits un, deux ou plusieurs composés choisis dans le groupe constitué par les lactones comportant au moins un groupe choisi parmi carboxyle, carboalcoxy, hydroxy et carboxylate, le mélange étant préparé après l'étape (iii), le mélange étant de préférence choisi dans le groupe constitué par les produits de raffinerie, les produits miniers et les produits ménagers,
- des mélanges réactionnels pour préparer des amides ou des esters desdits un, deux ou plusieurs composés choisis dans le groupe constitué par les lactones comportant au moins un groupe choisi parmi carboxyle, carboalcoxy, hydroxy et carboxylate, le mélange réactionnel étant préparé après l'étape (iii)
ou
(b) un ou plusieurs produits de réaction desdits un, deux ou plusieurs composés choisis dans le groupe constitué par les lactones comportant au moins un groupe choisi parmi carboxyle, carboalcoxy, hydroxy et carboxylate,
dans lequel ledit produit est choisi dans le groupe constitué par
- des produits comprenant un ou plusieurs polymères, de préférence des polyalcoxylates ou des polyesters, au moins l'un desdits polymères étant préparé à partir desdits un, deux ou plusieurs composés choisis dans le groupe constitué par les lactones comportant au moins un groupe choisi parmi carboxyle, carboalcoxy, hydroxy et carboxylate, dans une étape supplémentaire conduite après l'étape (iii),
- des solutions comprenant en tant que solvant ou constituant de solvant un ou plusieurs produits de réaction desdits un, deux ou plusieurs composés choisis dans le groupe constitué par les lactones comportant au moins un groupe choisi parmi carboxyle, carboalcoxy, hydroxy et carboxylate.

11. Acide tétrahydro-3-hydroxy-2-oxo-3-furanacétique.

12. Utilisation d'un, deux ou plusieurs composés choisis dans le groupe constitué par les acides tricarboxyliques aliphatiques, les esters de ceux-ci, les anhydrides de ceux-ci et les sels de ceux-ci, de préférence l'utilisation d'acide citrique, en tant que composé(s) de départ dans un procédé selon l'une quelconque des revendications 1 à 10 pour la préparation de
- composés choisis dans le groupe constitué par
- les lactones comportant un groupe carboxyle et un groupe hydroxy, de préférence choisies dans le groupe constitué par l'acide tétrahydro-3-hydroxy-5-oxo-3-furanacétique et l'acide tétrahydro-3-hydroxy-2-oxo-3-furanacétique
et
- les lactones comportant un groupe carboxyle et aucun groupe hydroxy, de préférence choisies dans le groupe constitué par l'acide tétrahydro-5-oxo-3-furanacétique et l'acide tétrahydro-2-oxo-3-furanacétique,
ou
- les mélanges de tels composés,
dans laquelle, de préférence, ces composés ou mélanges de tels composés sont préparés dans des quantités égales ou supérieures à 100 kg, plus préférablement égales ou supérieures à 500 kg, et encore plus préférablement égales ou supérieures à 1 000 kg par lot.

13. Utilisation d'un catalyseur d'hydrogénation hétérogène comprenant un ou plusieurs métaux choisis dans le groupe constitué par Co, Rh, Ir, Ni, Pd et Pt, en une quantité totale supérieure ou égale à 90 % en masse, de préférence supérieure ou égale à 95 % en masse, par rapport à la quantité totale du catalyseur d'hydrogénation hétérogène,
dans un procédé selon l'une quelconque des revendications 1 à 10 de préparation d'un produit comprenant un, deux ou plusieurs composés choisis dans le groupe constitué par les lactones comportant au moins un groupe choisi parmi carboxyle, carboalcoxy, hydroxy et carboxylate,
de préférence comprenant un, deux ou plusieurs composés choisis dans le groupe constitué par
- les lactones comportant un groupe carboxyle et un groupe hydroxy, de préférence choisies dans le groupe constitué par l'acide tétrahydro-3-hydroxy-5-oxo-3-furanacétique et l'acide tétrahydro-3-hydroxy-2-oxo-3-furanacétique
et
- les lactones comportant un groupe carboxyle et aucun groupe hydroxy, de préférence choisies dans le groupe constitué par l'acide tétrahydro-5-oxo-3-furanacétique et l'acide tétrahydro-2-oxo-3-furanacétique.

14. Utilisation selon la revendication 13, dans laquelle le catalyseur d'hydrogénation hétérogène comprend un ou plusieurs métaux choisis dans le groupe constitué par Rh et Pd.
